(19) Europäisches Patentamt
European Patent Office
Office européen des brevets

(11) Veröffentlichungsnummer: **0 095 640**
**B1**

(12) # EUROPÄISCHE PATENTSCHRIFT

(45) Veröffentlichungstag der Patentschrift: **29.01.86**

(51) Int. Cl.⁴: **C 07 D 417/12**, C 07 D 417/14, A 61 K 31/425

(21) Anmeldenummer: **83104793.1**

(22) Anmeldetag: **16.05.83**

(54) **Alkylenverbrückte Guanidinothiazolderivate, Verfahren zu ihrer Herstellung sowie ihre Verwendung als Arzneimittel.**

(30) Priorität: **28.05.82 DE 3220118**

(43) Veröffentlichungstag der Anmeldung: **07.12.83 Patentblatt 83/49**

(45) Bekanntmachung des Hinweises auf die Patenterteilung: **29.01.86 Patentblatt 86/5**

(84) Benannte Vertragsstaaten: **AT BE CH DE FR GB IT LI**

(56) Entgegenhaltungen:
**EP - A - 0 015 138**
**DE - A - 2 205 745**
**GB - A - 1 250 353**

(73) Patentinhaber: **BAYER AG, Konzernverwaltung RP Patentabteilung, D-5090 Leverkusen 1 Bayerwerk (DE)**

(72) Erfinder: **Ippen, Joachim, Dr., Morgengraben 14, D-5000 Koeln 80 (DE)**
Erfinder: **Perzborn, Elisabeth, Dr., Am Tescher Busch 13, D-5600 Wuppertal 11 (DE)**
Erfinder: **Puls, Walter, Dr., In den Birken 75, D-5600 Wuppertal 1 (DE)**
Erfinder: **Schaller, Klaus, Dr., Am Sonnenschein 38, D-5600 Wuppertal 1 (DE)**
Erfinder: **Seuter, Friedel, Dr., Moospfad 16, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 095 640 B1

**Beschreibung**

Die vorliegende Erfindung betrifft neue alkylenverbrückte Guanidinothiazolderivate, ein Verfahren zu ihrer Herstellung sowie ihre Verwendung zur Herstellung von Arzneimitteln, insbesondere solcher zur Beeinflussung des Fettstoffwechsels, sowie von Antithrombotika und von Antimykotika.

Gefunden wurden neue alkylenverbrückte Guanidinothiazolderivate der allgemeinen Formel I:

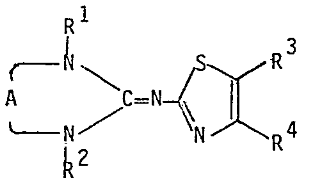

(I)

in welcher:

a) A für einen Ethylen-Rest,
$R^1$, $R^2$ und $R^3$ für Wasserstoff, und
$R^4$ für einen Methyl-, Chlormethyl-, Ethyloxycarbonylmethyl-, 5,6,7,8-Tetrahydro-2-naphthyl-, 4-Biphenylyl-, 4-Methoxyphenyl- und 4-Chlor-2-methylphenyl-Rest stehen, oder

b) A für einen Trimethylen-Rest,
$R^1$, $R^2$ und $R^3$ für Wasserstoff, und
$R^4$ für Wasserstoff, einen Methyl-, t.-Butyl-, Chlormethyl-, Ethyloxycarbonylmethyl-, 2,4-Dichlorphenoxymethyl-, Phenylthiomethyl-, 1-Imidazolylmethyl-, 1,2,4-Triazol-2-yl-methyl-, Phenyl-, 4-Cyclohexylphenyl, 1-Naphthyl-, 5,6,7,8-Tetrahydro-2-naphthyl-, 5-Indanyl-, 2,4-Dichlorphenyl-, 4-Biphenylyl-, 4-(4'-Chlor)-biphenylyl, 4-Chlor-2-methoxyphenyl-, 4-Hydroxyphenyl-, 4-Methoxyphenyl-, 4-Chlor-2-methylphenyl-, 4-(2',4'-Dichlor)-biphenylyl-, 4-Chlorphenyl-, 4-Nitrophenyl-, 2-Hydroxy-4-methoxyphenyl-, 5-Nitrofuran-2-yl, 2,6-Diethyl-4-hydroxyphenyl-, 4-Chlor-2-hydroxyphenyl-, 4-Aminophenyl-3-Acetylamino-4-chlor-2-methylphenyl-, 4-Chloracetyl-aminophenyl-, Adamantyl-, 1-Ethxyloxycarbonyl-2-(2'-6'-dichlorphenyl)-vinyl- und S-Isothioureidomethyl-Rest stehen, oder

c) A für einenTrimethylen-Rest,
$R^1$ und $R^2$ für Wasserstoff,
$R^4$ für Methyl, und
$R^3$ für einen Ethyloxycarbonyl-, Cyclohexyloxycarbonyl-, 2-Acetyloxyethyl- und 4-Chlorphenylcarboxamid-Rest stehen, oder

d) A für einen Trimethylen-Rest,
$R^1$ und $R^3$ für Wasserstoff,
$R^2$ für Methyl, und
$R^4$ für einen Methyl-, Phenyl-, 4-Chlor-2-methylphenyl- und 2,4-Dichlorphenyl-Rest stehen, oder

e) A für einen Tetramethylenrest,
$R^1$, $R^2$ und $R^3$ für Wasserstoff, und
$R^4$ für einen Methyl-, Phenyl-, 4-Chlor-2-methylphenyl- und 2,4-Dichlorphenyl-Rest stehen, oder

f) A für einen Trimethylenrest,
$R^1$ und $R^2$ für Wasserstoff, und
$R^3$ und $R^4$ für Phenyl stehen, oder

g) A für einen Trimethylenrest,
$R^1$, $R^2$ und $R^3$ für Wasserstoff, und
$R^4$ für einen $N^1$-Cyan-$N^2$-benzyl-$N^3$-phenyl-guanidin-Rest, einen $N^1$-Cyan-$N^2$-methyl-$N^3$-phenyl-guanidin-Rest und einen $N^1$-Cyan-$N^2$-phenyl-S-methyl-isothioharnstoff-Rest stehen und die Verknüpfung zum Thiazolring jeweils in der para-Position des Phenylrings erfolgt,

sowie deren physiologisch verträgliche Salze mit anorganischen und organischen Säuren und Basen.

Die Synthese der erfindungsgemässen alkylenverbrückten Guanidinothiazolderivate der allgemeinen Formel I erfolgt gemäss folgendem Schema:

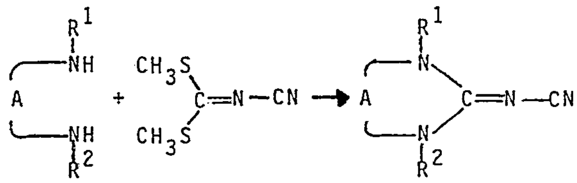

(II) (III) (IV)

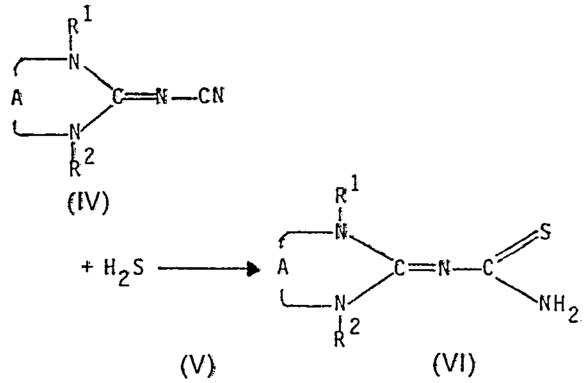

(IV) (V) (VI)

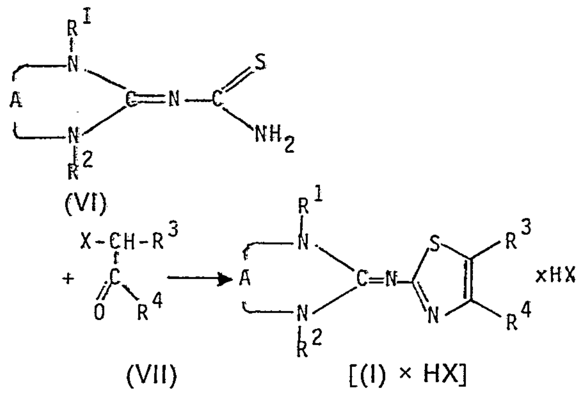

(VI) (VII) [(I) × HX]

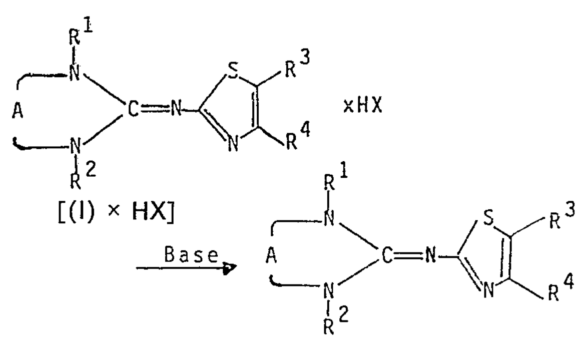


[(I) × HX] (I)

Die im Formelschema angegebenen Abkürzungen wie A, $R^1$, $R^2$, $R^3$ und $R^4$ haben die vorstehend angegebene Bedeutung; X bedeutet Hydroxy oder Halogen, vorzugsweise Chlor oder Brom.

In an sich bekannter Weise [vgl. C.M. Baltzer und C.G. MacCarty, J. Org. Chem. *38*, 155 (1973); DT-OS Nr. 2205745] erhält man die cyclischen N-Cyanguanidine der allgemeinen Formel IV durch Umsetzung von Alkylendiaminen II mit N-Cyanimidodithiokohlensäure-S,S$^1$-dimethylester III.

Durch Addition von Schwefelwasserstoff an N-Cyanguanidin erhält man Guanylthioharnstoff [vgl. Org. Synth. Coll. Vol. IV 502]. In analoger Weise erhält man die N,N$^1$-Alkylenverbrückten Guanylthioharnstoffe VI aus den cyclischen N-Cyanguanidinen IV. Als Lösungsmittel dienen hierbei Wasser sowie protische und aprotische organische Lösungsmittel, beispielsweise Wasser, Ethanol, Dimethylformamid oder Pyridin bzw. Gemische der Lösungsmittel. Die Reaktion wird bei einer Temperatur von 0-150°C durchgeführt, bevorzugt bei 20-120°C.

Die Umsetzung von α-Halogenketonen mit Thiocarboxamiden zu 1,3-Thiazolen ist prinzipiell bekannt [vgl. C. Ferri, Reaktionen der organischen Synthese, Thieme Verlag, Stuttgart 1978, S. 731]. Durch Reaktion der N,N$^1$-Alkylenverbrückten Guanylthioharnstoff VI mit α-Halogenketonen und -aldehyden VII erhält man die erfindungsgemässen alkylenverbrückten Guanidinothiazolderivate [(I) × HX] bzw. I.

Als Verdünnungsmittel bei dieser Reaktion werden organische Lösungsmittel sowie Wasser eingesetzt. Bevorzugte Lösungsmittel sind Wasser, aromatische und heteroaromatische Kohlenwasserstoffe, hiervon besonders bevorzugt Toluol, Pyridin, Ketone z. B. Aceton und Alkohole, z. B. Ethanol.

Die Reaktionstemperaturen können in einem grösseren Bereich variiert werden. Allgemein arbeitet man zwischen etwa 0 bis 180°C, vorzugsweise zwischen Raumtemperatur und der Siedetemperatur des jeweiligen Verdünnungsmittels.

Die Reaktionsdauer ist von der Temperatur und dem Verdünnungsmittel abhängig und liegt zwischen 0,5 und 24 h.

Aus den Salzen der erfindungsgemässen Verbindungen [(I) × HX] können die freien alkylenverbrückten Guanidinothiazolderivate I mit Basen freigesetzt werden. Bevorzugt eingesetzt werden Alkali- und Erdalkalikarbonate, -hydrogenkarbonate und -hydroxide sowie Ammoniak und organische Basen wie tertiäre aliphatische und aromatische Amine. Als Verdünnungsmittel wird bevorzugt Wasser eingesetzt.

Es ist klar, dass die alkylenverbrückten Guanidinothiazolderivate der allgemeinen Formel I unter der Voraussetzung, dass $R^1$ und $R^2$ Wasserstoff bedeuten, auch als Doppelbindungsisomere, die durch die Formeln Ia und Ib dargestellt werden, vorliegen können.

Diese Isomeriemöglichkeiten können auch bei den jeweiligen Vorstufen vorliegen. Die vorliegende Erfindung umfasst auch die isomeren Formen Ia und Ib der allgemeinen Formel:

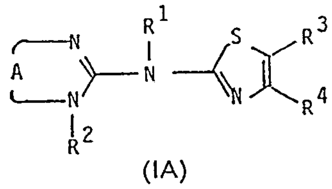

(IA)

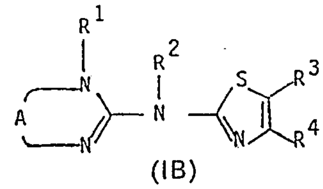

(IB)

Beispiele für die neuen Wirkstoffe sind die im einzelnen in Tabelle 1 aufgeführten Verbindungen.

Aus GB-A Nr. 1250353 sind 2-Amino-5-nitrothiazole bekannt geworden, die antibakterielle Wirkung haben. Die erfindungsgemässen Verbindungen enthalten jedoch keine Nitrogruppen.

EP-A1 Nr. 0015138 beschreibt Guanidinothiazole, in denen die Guanidino-Einheit im Gegensatz zu den erfindungsgemässen Verbindungen nicht verbrückt ist, und die Histamin-$H_2$-antagonisten sind.

Alle diese bekannten Verbindungen haben keine Wirkungen auf den Arachidon-Metabolismus-Zyklus.

Die erfindungsgemässen Verbindungen stellen somit eine Bereicherung der Pharmazie dar.

*Tabelle I*

| Beispiel | A | $R^1$ | $R^2$ | $R^3$ | $R^4$ | HX | Reaktionsbedingungen Verdünnungsmittel | Temperatur | Zeit (h) | Ausbeute (%) | Fp (°C) | Herstellung der freien Verbindungen aus den Salzen |
|---|---|---|---|---|---|---|---|---|---|---|---|---|
| 2 | $-(CH_2)_2-$ | H | H | H | $-CH_2Cl$ | HCl | Aceton | Raumtemp. | 10 | 91 | 228-230 | |
| 3 | $-(CH_2)_2-$ | H | H | H | $-CH_2CO_2C_2H_5$ | — | Aceton | Rückfl. | 5 | 29 | 124-125 | $NaHO_3,H_2O$ |
| 4 | $-(CH_2)_2-$ | H | H | H | 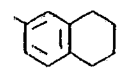 | — | Aceton | Rückfl. | 2 | 15 | 228-230 | $NaOH,H_2O$ |
| 5 | $-(CH_2)_2-$ | H | H | H | 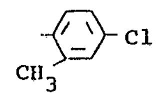 | — | Aceton | Rückfl. | 4 | 34 | 230-231 | $NaOH,H_2O$ |
| 6 | $-(CH_2)_2-$ | H | H | H | 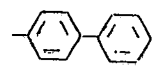 | — | Aceton | Rückfl. | 4 | 44 | 279-280 | $NH_3,H_2O$ |
| 7 | $-(CH_2)_2-$ | H | H | H | 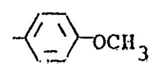 | — | Aceton | Rückfl. | 2 | 33 | 260-261 | — |
| 8 | $-(CH_2)_3-$ | H | H | H | H | $H_2O$ | Wasser | Raumtemp. | 3 | 25 | 117-118 | $NH_3,H_2O$ |
| 9 | $-(CH_2)_3-$ | H | H | H | $-CH_3$ | HCl | Aceton | Raumtemp. | 8 | 90 | 250-251 | |
| 10 | $-(CH_2)_3-$ | H | H | H | $-C(CH_3)_3$ | HCl | Aceton | Rückfl. | 10 | 92 | 199-201 | |
| 11 | $-(CH_2)_3-$ | H | H | H | $-CH_2Cl$ | HCl | Aceton | Rückfl. | 2 | 72 | 221-223 (Zers.) | |
| 12 | $-(CH_2)_3-$ | H | H | H | 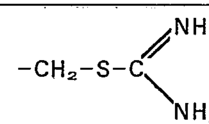 | 2HCl | | | | 71 | 228-238 | |
| 13 | $-(CH_2)_3-$ | H | H | H | $-CH_2-CO_2C_2H_5$ | — | Aceton | Rückfl. | 5 | 41 | 168-169 | $NaHCO_3,H_2O$ |
| 14 | $-(CH_2)_3-$ | H | H | $-CO_2C_2H_5$ | $-CH_3-$ | — | Aceton | Rückfl. | 2 | 22 | 185-186 | $NaHCO_3,H_2O$ |
| 15 | $-(CH_2)_3-$ | H | H | 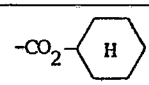 | $-CH_3-$ | — | Aceton | Rückfl. | 4 | 27 | 187-188 | $NaHCO_3,H_2O$ |
| 16 | $-(CH_2)_3-$ | H | H | $-(CH_2)_2-OAC$ | $-CH_3$ | HCl | Aceton | Rückfl. | 10 | 8 | 230-231 | |
| 17 | $-(CH_2)_3-$ | H | H | $-CO-NH-$ 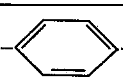 $-Cl$ | $-CH_3$ | HCl | Aceton | Rückfl. | 1 | 92 | 289-290 | |

*Tabelle I* (Fortsetzung)

| Bei-spiel | A | R¹ | R² | R³ | R⁴ | HX | Reaktionsbedingungen | | | Aus-beute (%) | Fp (°C) | Herstellung der freien Verbindungen aus den Salzen |
|---|---|---|---|---|---|---|---|---|---|---|---|---|
| | | | | | | | Verdünnungs-mittel | Temperatur | Zeit (h) | | | |
| 18 | $-(CH_2)_3-$ | H | H | H | 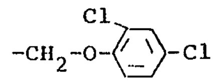 | — | | | | 9 | 185-187 | |
| 19 | $-(CH_2)_3-$ | H | H | H | 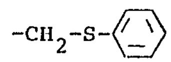 | — | | | | 46 | 139-143 | |
| 20 | $-(CH_2)_3-$ | H | H | H | 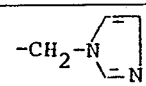 | — | | | | 29 | 163-164 | |
| 21 | $-(CH_2)_3-$ | H | H | H | 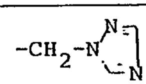 | — | | | | 19 | 206-208 | |
| 22 | $-(CH_2)_3-$ | H | H | H | 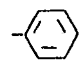 | HCl | Aceton | Rückfl. | 1 | 90 | 244-245 | |
| 23 | $-(CH_2)_3-$ | H | H | H | 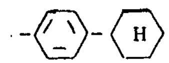 | — | Aceton | Rückfl. | 2 | 42 | 270-272 | $NaOCH_3$ $C_2H_5OH$ |
| 24 | $-(CH_2)_3-$ | H | H | H | 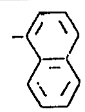 | — | Aceton | Rückfl. | 2 | 49 | 223-228 | $H_2O, NaHCO_3$ |
| 25 | $-(CH_2)_3-$ | H | H | H | 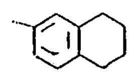 | HCl | Aceton | Rückfl. | 2 | 66 | 230-232 | |
| 26 | $-(CH_2)_3-$ | H | H | H | 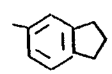 | HCl | $C_2H_5OH$ | Rückfl. | 2 | 75 | 234-236 | |
| 27 | $-(CH_2)_3-$ | H | H | H | 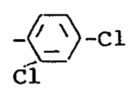 | HCl | Aceton | Rückfl. | 3 | 77 | 255-257 | |
| 28 | $-(CH_2)_3$ | H | H | H | 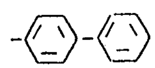 | HCl | Aceton | Rückfl. | 2 | 70 | 232-233 | |

*Tabelle I* (Fortsetzung)

| Bei-spiel | A | $R^1$ | $R^2$ | $R^3$ | $R^4$ | HX | Reaktionsbedingungen Verdünnungs-mittel | Temperatur | Zeit (h) | Aus-beute (%) | Fp (°C) | Herstellung der freien Verbindungen aus den Salzen |
|---|---|---|---|---|---|---|---|---|---|---|---|---|
| 29 | $-(CH_2)_3-$ | H | H | H | 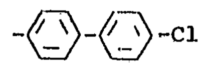 | HCl | Aceton | Rückfl. | 3,5 | 13 | 279-281 (Zers.) | |
| 30 | $-(CH_2)_3-$ | H | H | H | 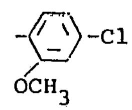 | HCl | Aceton | Rückfl. | 2 | 43 | 236-237 | |
| 31 | $-(CH_2)_3-$ | H | H | H | 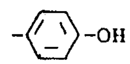 | HCl | Aceton | Rückfl. | 4 | 89 | 280-283 | |
| 32 | $-(CH_2)_3-$ | H | H | H | 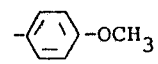 | HCl | Aceton | Rückfl. | 4 | 63 | 224-225 | |
| 33 | $-(CH_2)_3-$ | H | H | H | 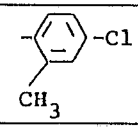 | HCl | Aceton | Rückfl. | 2 | 81 | 242-244 | |
| 34 | $-(CH_2)_3-$ | H | H | H | 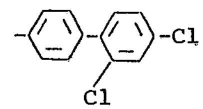 | HCl | Aceton | Rückfl. | 2 | 66 | 208-210 | |
| 35 | $-(CH_2)_3-$ | H | H | H | 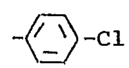 | HBr | Aceton | Rückfl. | 2 | 77 | 263-265 | |
| 36 | $-(CH_2)_3-$ | H | H | H | 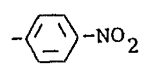 | HBr | Aceton | Rückfl. | 2 | 97 | 308-309 (Zers.) | |
| 37 | $-(CH_2)_3-$ | H | H | H | 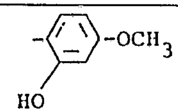 | — | Pyridin | Rückfl. | 2 | 63 | 210-211 | |
| 39 | $-(CH_2)_3-$ | H | H | H | 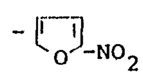 | HBr | Aceton | Rückfl. | 1 | 92 | > 310 | |

*Tabelle I* (Fortsetzung)

| Beispiel | A | R¹ | R² | R³ | R⁴ | HX | Reaktionsbedingungen: Verdünnungsmittel | Reaktionsbedingungen: Temperatur | Reaktionsbedingungen: Zeit (h) | Ausbeute (%) | Fp (°C) | Herstellung der freien Verbindungen aus den Salzen |
|---|---|---|---|---|---|---|---|---|---|---|---|---|
| 40 | $-(CH_2)_3-$ | H | H | H | 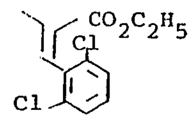 | — | Aceton | Rückfl. | 3 | 59 | 225-226 | $H_2O,NH_3$ |
| 41 | $-(CH_2)_3-$ | H | H | $-CH_3$ | $-CH=N-NH-$ 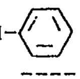 | — | Aceton | Rückfl. | 5 | 17 | 232-236 | $H_2O,NaHCO_3$ |
| 42 | $-(CH_2)_3-$ | H | H | H | 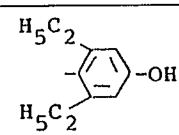 | HCl | Aceton | Rückfl. | 2 | 71 | 236-238 | |
| 43 | $-(CH_2)_3-$ | H | H | H | 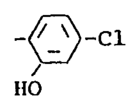 | — | Pyridin | Rückfl. | 3 | 90 | >320 | |
| 44 | $-(CH_2)_3-$ | H | H | H | 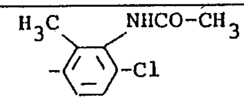 | — | Aceton | Rückfl. | 2 | 19 | 275-277 | $H_2O,NaHCO_3$ |
| 45 | $-(CH_2)_3-$ | H | H | H | 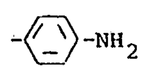 | — | Aceton | Rückfl. | 2 | 55 | 238-240 | $H_2O,NaHCO_3$ |
| 46 | $-(CH_2)_3-$ | H | H | H | 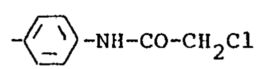 | HCl | Aceton | Rückfl. | 2 | 55 | 241 (Zers.) | |
| 47 | $-(CH_2)_3-$ | H | H | 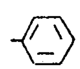 | 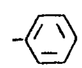 | — | Pyridin | Rückfl. | 4 | 54 | 249-250 | |
| 48 | $-(CH_2)_3-$ | H | $CH_3$ | H | $-CH_3$ | — | Aceton | Rückfl. | 2 | 73 | 90- 91 | $H_2O,NaOH$ |
| 49 | $-(CH_2)_3-$ | H | $CH_3$ | H | 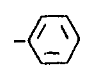 | HCl | Aceton | Rückfl. | 2 | 95 | 282-283 | |
| 50 | $-(CH_2)_3-$ | H | $CH_3$ | H | 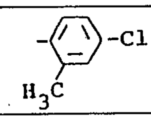 | HCl | Aceton | Rückfl. | 1 | 45 | 215-216 | |

*Tabelle I* (Fortsetzung)

| Beispiel | A | $R^1$ | $R^2$ | $R^3$ | $R^4$ | HX | Reaktionsbedingungen: Verdünnungsmittel | Reaktionsbedingungen: Temperatur | Reaktionsbedingungen: Zeit (h) | Ausbeute (%) | Fp (°C) | Herstellung der freien Verbindungen aus den Salzen |
|---|---|---|---|---|---|---|---|---|---|---|---|---|
| 51 | $-(CH_2)_3-$ | H | $CH_3$ | H | 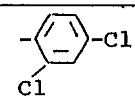 | HCl | Aceton | Rückfl. | 1 | 68 | 247-249 | |
| 52 | $-(CH_2)_4-$ | H | H | H | $-CH_3$ | HCl | Aceton | Rückfl. | 2 | 53 | 185-187 | |
| 53 | $-(CH_2)_4-$ | H | H | H | 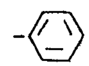 | HCl | Aceton | Rückfl. | 2 | 78 | 235-237 | |
| 54 | $-(CH_2)_4-$ | H | H | H | 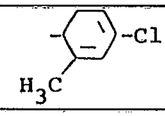 | HCl | Aceton | Rückfl. | 2 | 66 | 196-197 | |
| 55 | $-(CH_2)_4-$ | H | H | H | 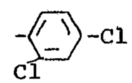 | HCl | Aceton | Rückfl. | 2 | 77 | 228-230 | |
| 56 | $-(CH_2)_3-$ | H | H | H | 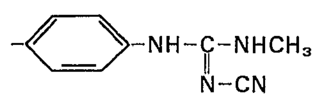 | — | | | | 49 | 283-285 | |
| 57 | $-(CH_2)_3-$ | H | H | H | 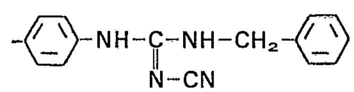 | | | | | 42 | 236-237 | |
| 58 | $-(CH_2)_3-$ | H | H | H | 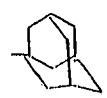 | — | Aceton Pyridin | Rückfl. | 3 | 52 | 257-258 | $H_2O,NH_3$ |
| 59 | $-(CH_2)_3-$ | H | H | H | 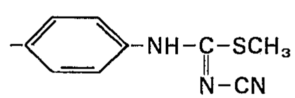 | | | | | 56 | >310 | |

Die Verbindungen der Beispiele 18, 19, 20, 21 der Tabelle I lassen sich auch aus der Verbindung des Beispiels 11 durch Umsetzung mit entsprechenden Nucleophilen wie 2,4-Dichlorphenol, Thiophenol, Imidazol und Triazol herstellen.

Die Substanzbeispiele 56 und 57 sind auch ausgehend von der Verbindung des Beispiels 45 durch Umsetzung mit z. B.

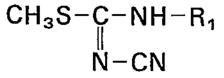

wobei $R_1$ = Alkyl oder Aryl bedeutet, erhältlich.

Die biologische Wirkung der erfindungsgemäss hergestellten Verbindungen wurde durch folgende Experimente nachgewiesen:

*Thrombozytenaggregationshemmende Wirkung*

Die Verwendung thrombozytenaggregationshemmender Medikamente als Antithrombotika kann mittlerweile als anerkanntes Therapieprinzip betrachtet werden. Die Beteiligung der Thrombozyten bei atherosklerotischen Prozessen ist ebenfalls bekannt, so dass Substanzen mit diesen Eigenschaften, die in den genannten Indikationen verwendet werden sollen, als günstig zu beurteilen sind.

Für die *in vitro*-Versuche wurde Blut von gesunden Probanden beiderlei Geschlechts verwendet. Als Antikoagulans wurde ein Teil 3,8%ige wässerige Natriumzitratlösung 9 Teilen Blut zugemischt. Mittels Zentrifugation erhält man aus diesem Blut plättchenreiches Zitratplasma (PRP) (Jürgens/Beller: Klinische Methoden der Blutgerinnungsanalyse; Thieme-Verlag Stuttgart 1959).

Für die *in vitro*-Versuche wird die minimal effektive Wirkstoffkonzentration angegeben, die in den entsprechenden PRP-Proben die Thrombozytenaggregation hemmt. Für diese Untersuchungen wurden 0,8 ml PRP und 0,1 ml der Wirkstofflösung bei 37°C im Wasserbad vorinkubiert. Anschliessend wurde die Thrombozytenaggregation nach der turbidometrischen Methode (Born, G.V.R.: J. Physiol. *162*, 67, 1962) im Aggregometer bei 37°C bestimmt (Seuter, F.: Haemostasis *5*, 85-95, 1976). Hierzu wurde die vorinkubierte Probe mit 0,1 ml einer aggregationsauslösenden Kollagensuspension versetzt.

Die Veränderung der optischen Dichte in der Probe des PRP wurde während einer Zeitdauer von 6 min aufgezeichnet und über den Ausschlag nach 6 min bestimmt. Die prozentuale Hemmung errechnet sich aus den entsprechenden Ausschlägen. Weiterhin wurde die Thrombozytenaggregation im Rahmen des Thromboatherosklerose-Versuchs mitbestimmt. Bei der Durchführung dieser Versuche wurde den Tieren die Wirksubstanz in einer Tylosesuspension oral während 10 d verabreicht. Am 10. Tag wurden die Tiere 90 min nach der oralen Verabreichung der Substanz entblutet und das PRP mittels Zentrifugation gewonnen. Nach Gewinnung des PRP beginnt *in vitro* die Messung der Aggregationshemmung analog dem Verfahren, welches für die *in vitro*-Versuche beschrieben ist; jedoch ohne Vorinkubation der Proben. 3 Teile Ratten-PRP wurden ausserdem mit einem Teil physiologischer Kochsalzlösung verdünnt.

*Anti-atherosklerotische Wirkung*

Herz- und Kreislauferkrankungen führen in den westlichen Industrieländern mit > 50% der Todesfälle die Sterbestatistiken an. Die praktische und soziale Bedeutung ergibt sich aus der Häufigkeit und Schwere der Krankheiten, die in hohem Masse Menschen auf der Höhe ihrer Leistungsfähigkeit befallen. Im arteriellen Teil des Gefässsystems handelt es sich grösstenteils um chronische Krankheitsprozesse, vorwiegend arteriosklerotische Veränderungen, die meistens zu einem teilweisen oder vollständigen Verschluss des Gefässes führen. Die durch Minderdurchblutung entstehenden Gewebeschäden können bis zum lokalen Gewebstod, dem Infarkt (Herz, Gehirn) führen. Die Coronarsklerose z. B. steht im Mittelpunkt der Infarktpathogenese.

Arteriosklerose bzw. Atherosklerose im engeren Sinne ist eine sehr komplexe Erkrankung, bei der wir die Beteiligung von mindestens 3 Hauptprozessen beobachten können: 1. Gefässwandschädigung mit nachfolgender Proliferation ihrer glatten Muskelzellen und Entstehung einer fibromuskuloelastischen Läsion; 2. Lipideinlagerung; 3. Murale Thrombenbildung und deren Einlagerung in die Gefässwand. Diesen Faktoren wurde in der Vergangenheit mit unterschiedlicher Wertigkeit Rechnung getragen, was zu der „thrombogenen" und „Lipidinfiltrations-theorie" geführt hat.

In unserem Experiment haben wir beide Faktoren berücksichtigt, wobei jedoch die Gefässwandschädigung mit nachfolgender Intimaproliferation im Vordergrund steht. Diese Vorgänge werden in der Ätiopathogenese der Arteriosklerose in neuerer Zeit von vielen Autoren als die bedeutsamsten angesehen.

Aufgrund mancher fliessender Übergänge zwischen Atherosklerose und Thrombose haben wir die verwendete Methode „Thromboatherosklerose-Modell" genannt.

*Methodik*

Lit.: Seuter, F., Sitt, R. und Busse, W.D., Experimentally induced thromboatherosclerosis. Folia angiologica *28*, 85 (1980).

Durch Kombination zweier Noxen (Risikofaktoren), nämlich Gefässläsion und Hyperlipämie werden bei Ratten atherosklerotische Plaques induziert. Durch Unterkühlung der Arteria carotis comm. (2 min, −10°C) kommt es zu einer partiellen bzw. vollständigen Endothelabschilferung in einem lokal begrenzten Bereich. Intimaproliferationen treten in einem für derartige Versuche relativ kurzen Zeitraum von 10 d auf und entsprechen weitgehend den Frühstadien atherosklerotischer Plaques beim Menschen. Die Proliferationszonen werden abpräpariert und gewogen. Das Feuchtgewicht beträgt in dem unterkühlten Segment von 1 cm ca. 200-300 µg. Den Tieren wird zusätzlich eine fettreiche Diät der folgenden Zusammensetzung verabreicht:

3% Chloesterin
1% Na-cholat
34% Rohrzucker
10% Schweineschmalz
52% Normalfutter (Altromin®).

Die Fetteinlagerungen werden mit dem fettlöslichen Farbstoff Sudan IV angefärbt und können als zusätlicher Parameter bestimmt werden.

Neben alkylierend wirkenden Zytostatika und Glucocorticoiden, die unspezifisch Zellproliferationen hemmen, war bisher nur Heparin nach parenteraler Verabreichung in diesem Modell wirksam. Lipidsenkende Substanzen wie Chloestyramin, Neomycin, Nikotinsäure etc., waren unwirksam. Die dosisabhängige Wirkung von Heparian, das in allen Versuchen als positiver Referenzstandard dient, wurde beschrieben (Sitt, R., Seuter, F. und Busse, W.D., Suppression by Heparin of intimal proliferation in the injured carotid artery (rat). Arch. Pharmacol., Suppl. *311*, Abstr. 188, 1980).

Überraschenderweise waren Verbindungen aus der Chemischen Substanzklasse der alkylenverbrückten Guanidinothiazolderivate nach oraler Verabreichung antiatherosklerotisch wirksam, indem die Bildung eines myointimalen Plaques (Intimaproliferation) gehemmt wurde (Tab.).

*Arachidonsäure-Metabolismus*

Die Erkenntnis, dass eine Reihe von Metaboliten der Arachidonsäure für die Ätiologie, Pathogenese und das Manifestwerden von Coronarerkrankungen, wie Angina pectoris (Prinzmetal), Herzinfarkt, Erkrankungen der peripheren Gefässe, Arteriosklerose bedeutsam sind, hat dazu geführt, dass die pharmakologische Beeinflussung des Arachidonsäurestoffwechsels ein wichtiger Ansatzpunkt für die Therapie von Kreislauferkrankungen geworden ist.

Einzelne Metaboliten der Arachindonsäure sind sehr potente Regulatoren der Gefässfunktionen und haben darüber hinaus erhebliche Bedeutung für die Entstehung von Thromboembolien und pathologischen Veränderungen der Gefässwand.

Die physiologische bzw. pathophysiologische Bedeutung pharmakologischer Eingriffe in den Arachidonsäurestoffwechsel wird entscheidend von der Art und dem Ort des Eingriffes in den Arachidonsäurestoffwechsel beeinflusst. Sehr vereinfacht lassen sich die Verhältnisse etwa wie folgt darstellen:

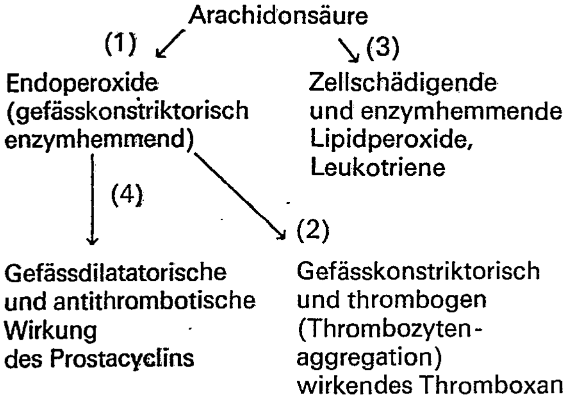


(1) = Cyclooxygenasehemmer, die die Bildung von Endoperoxiden und damit von Thromboxan unterdrücken
(2) = Thromboxansynthetasehemmer
(3) = Lipoxygenasehemmer
(4) = Prostacyclin-Stimulatoren

Aus dem Schema wird deutlich, dass die Therapie von Gefässerkrankungen durch Eingriff in den Arachidonsäurestoffwechsel besonders wirkungsvoll durch Substanzen durchgeführt werden kann, die nicht nur an einer Stelle in den Stoffwechsel der Arachidonsäure eingreifen, sondern ein Profil von hemmenden oder fördernden Effekten auf die einzelnen Stoffwechselschritte haben.

Die über den Lipoxygenaseabbauweg entstehenden Lipidperoxide sind bedeutsam bei Entzündungsreaktionen. Es gibt jedoch Anhaltspunkte, dass diese Peroxide auch bei der Entstehung von Thrombosen und Endothelschäden beteiligt sind. Blockade des Lipoxygenaseweges des Arachidonsäuremetabolismus könnte demzufolge eine weitere wünschenswerte Eigenschaft sein.

Bestimmte Lipidperoxide, wie HPETE wirken allgemein zellschädigend und scheinen (irreversibel) Kontrakturen von glatten Muskelzellen (Gefässen) hervorzurufen.

*Methodik:*

$^3$H-Arachidonsäure wird in Humanthrombozyten in die Produkte des Cyclooxygenase-/Thromboxansynthetaseweges, insbesondere Thromboxan $A_2$, und die des Lipoxygenaseweges, HETE, umgesetzt. Die dünnschichtchromatographische Auftrennung der entstehenden Produkte liefert ein charakteristisches Bild. Hemmer der einzelnen enzymatischen Reaktionen verändern dieses Bild in typischer Weise. (Bailey, J.M. *et al.*, Prostaglandins *13*, 379-492 (1977).) Die Wirkung der erfindungsgemässen Verbindungen auf einzelne Reaktionen des Arachidonsäurestoffwechsels sind in der Tabelle 2 eingetragen.

Aufgrund der genannten Wirkqualitäten eignen sich die erfindungsgemässen Verbindungen beispielsweise zur Behandlung oder Verhinderung von Herzinfarkten, der Angina pectoris, thromboembolischer Erkrankungen im venösen und arteriellen Bereich (postoperativ, transitorisch ischämische Attacken, Amaurosis fugax etc.), anderer vaskulärer Krankheitsbilder wie der Arteriosklerose.

*(Tabelle auf der nächsten Seite)*

*Antimykotische Wirkung*

Die erfindungsgemässen alkylenverbrückten Guanidinothiazole zeigen eine überraschende antifungische Wirkung gegen Dermatophyten, Hefen und Schimmelpilze.

Verglichen mit den Konkurrenzpräparaten „Ketoconazol" und „Doconazol" zeigen die beanspruchten Substanzen *in vitro* eine vergleichbare und gegenüber ausgewählten Keimen sogar eine bessere Hemmwirkung (Minimale Hemmkonzentration).

*Tabelle der Wirkungen*

| Beispiel | Biologische Wirkung | Wirksamkeit, $ED_{50}$ (Dosis-/Konzentrationsbereich) |
|---|---|---|
| 13 | TAH in vitro | $1 \times 10^{-4}$–$1 \times 10^{-5}$ g/ml |
| 15 | Cyclo-oxygenasehemmung | $1 \times 10^{-5}$–$3 \times 10^{-6}$ g/ml |
| 16 | TAH in vitro | $1 \times 10^{-4}$–$1 \times 10^{-5}$ g/ml |
| 17 | TAH ex vivo (Atherosklerose-Versuch) | 100 mg/kg p.o. |
| 25 | TAH ex vivo | 100 mg/kg p.o. |
| 49 | a) anti-atherosklerotisch<br>b) TAH ex vivo<br>c) Cyclo-oxygenasehemmung | 100 mg/kg p.o.<br>100 mg/kg p.o.<br>$1 \times 10^{-5}$ g/ml |
| 53 | TAH in vitro | $1 \times 10^{-4}$–$1 \times 10^{-5}$ g/ml |
| TAH=Thrombocytenaggregationshemmung | | |

*Test der antimykotischen* in vitro- *Wirksamkeit*

Versuchsbeschreibung:

Die *in vitro*-Prüfungen wurden im Reihenverdünnungstest mit Keiminokula von durchschnittlich $5 \times 10^4$ Keimen/ml Substrat durchgeführt. Als Nährmedium dienten:

a) für Dermatophyten und Schimmelpilze: Sabourands' milieu d'épreuve

b) für Hefen: Fleischextrakt-Traubenzucker-Bouillon.

Die Bebrütungstemperatur betrug 20° C, die Bebrütungsdauer lag bei 24 bis 96 s bei Hefen und 96 s bei Dermatophyten und Schimmelpilzen.

Die überraschende Wirkung der erfindungsgemässen Stoffgruppe sei beispielhaft durch die folgenden Testergebnisse belegt:

| Beispiele | Minimale Hemmkonzentrationen (mcg/ml) | | | | |
|---|---|---|---|---|---|
| | Trichophyton mentagrophytes | Microsporum canis | Candida albicans | Torulopsis glabrata | Aspergillus fumigatus |
| 22 | <1 | <1 | 4 | 32 | < 1 |
| 24 | <1 | <1 | 2 | 8 | < 1 |
| 27 | <1 | <1 | <1 | 16 | 8 |
| 33 | 2 | 2 | 2 | 16 | 2 |
| | | | | | |
| Ketoconazol | <1 | 4 | <1 | 1 | 16 |
| | | | | | |
| Doconazol | <1 | 8 | <1 | < 1 | 4 |
| | | | | | |

*Cholesterol-Belastung:*

Cholesterolbelastungstests wurden an nüchternen männlichen oder weiblichen Wistar-Ratten mit einem Körpergewicht zwischen 130-230 g durchgeführt.

Cholesterol und die zu prüfende Substanz wurden in 0,75%iger Traganth-Suspension gleichzeitig oral appliziert. Nach 24 h wurden die Tiere getötet und der Cholesterolgehalt in der Leber bestimmt. Cholesterol wurde nach der Liebermann-Burchard-Methode mit der Testcombination von Boehringer Mannheim bestimmt. Kontrolltiere erhielten ein entsprechendes Volumen Cholesterol-Traganth-Suspension ohne Testsubstanz. Zur Kontrolle des Cholesterol-Anstiegs als Folge der Cholesterol-Belastung wurde einer weiteren Kontrollgruppe Traganth-Suspension ohne Cholesterol oral appliziert.

Die überraschende Wirkung der erfindungsgemässen Stoffgruppe sei beispielhaft durch die folgenden Testergebnisse belegt:

| Beispiel Nr. | Dosis (mg/kg) p.o. | Verminderung des Lebercholesterolanstiegs im Vergleich zur Kontrolle nach oraler Cholesterolbelastung (in %) |
|---|---|---|
| 17 | 100 | 56,1 |
| 25 | 10 | 65,3 |
| | 30 | 78,1 |

Es ist als ausgesprochen überraschend zu bezeichnen, dass die erfindungsgemässen Verbindungen diese neuen und vorteilhaften Wirkungen besitzen. Als neuartige Stoffklasse zur Behandlung von Stoffwechselstörungen, bei gleichzeitig sehr guter Verträglichkeit, stellen sie eine Bereicherung der Pharmazie dar.

*Herstellungsbeispiele:*

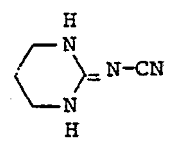
(a)

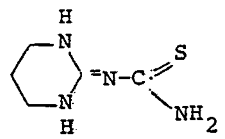
(b)

*2-(N-Thiocarbamoylimino)-hexahydropyrimidin (b)*

In eine Lösung von 190 g (1,53 mol) 2-(N-Cyanimino)-hexahydropyrimidin (a) in 760 ml Wasser leitet man bei 75°C zuerst 12 h Schwefelwasserstoff ein; danach bei 65-70°C weitere 25-30 h. (Am zweiten Tag geht ein Teil des Niederschlages nicht mehr in Lösung.) Anschliessend wird die Suspension auf 45°C erwärmt und 46 ml 40%ige NaOH zugegeben. Man rührt 30 min nach, vermahlt den Rückstand und saugt ab. Nach dem Trocknen erhält man 228 g (94%) Produkt. Fp = 187-189°C (Zers.).

*Analyse:*

Berechnet: C 37,97 H 6,37 N 35,43%
Gefunden: C 38,1 H 6,3 N 35,4 %

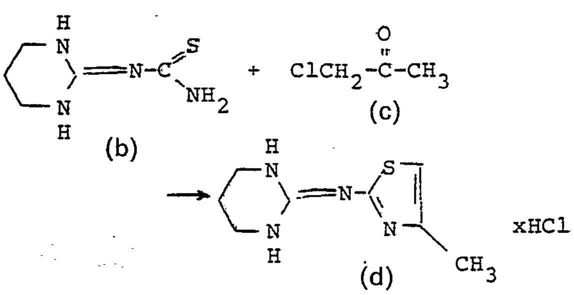
(b) (c) (d)

*2-(N-(4'-Methylthiazol-2'-yl)imino)-hexahydropyrimidinhydrochlorid (d)*

Zu 7,9 g (0,05 mol) 2-(N-Thiocarbamoyliminο)-hexahydropyrimidin (b) in 50 ml Aceton tropft man eine Lösung von 4,62 g (0,05 mol) α-Chloraceton (c) in 20 ml Aceton. Man rührt 8 h bei Raumtemperatur, lässt über Nacht stehen und saugt den Niederschlag ab. Das Produkt wird mit Aceton gewaschen und anschliessend getrocknet.

Ausbeute: 10,4 g (90%), Fp = 250-251°C.

*Analyse:*

Berechnet: C 41,29 H 5,59 Cl 15,26 N 24,08%
Gefunden: C 41,1 H 5,2 Cl 15,0 N 24,2 %

Die Verbindung (d) entspricht dem alkylenverbrückten Guanidinothiazol (9) der Tabelle I.

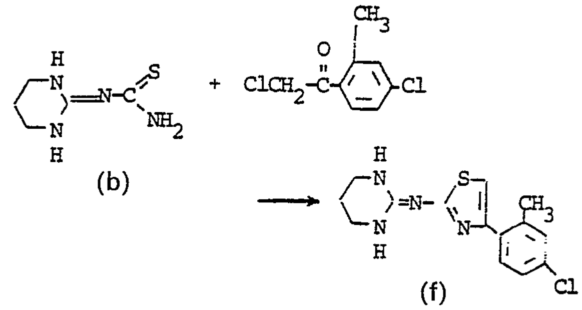
(b) (f)

*2-(N-(4'-(4"-Chlor-2"-methylphenyl)-thiazol-2'-yl)-imino)-hexahydropyrimidin-hydrochlorid (f)*

Zu 15,8 g (0,1 mol) 2-(N-Thiocarbamoylimino)-hexahydropyrimidin (b) in 100 ml Aceton gibt man 20,3 g (0,1 mol) α,4-Dichlor-2-methylacetophenon (e) und erhitzt 2 h zum Sieden. In der Siedehitze erhält man eine Lösung, aus der nach ca. 10 min das Produkt ausfällt. Man kühlt das Reaktionsgemisch ab, saugt ab und kristallisiert das Produkt aus Ethanol um.

Ausbeute: 27,7 g (81%), Fp = 242-244°C.

*Analyse:*

Berechnet:
C 48,98 H 4,66 Cl 20,7 N 16,33 S 9,33%
Gefunden:
C 49,1 H 4,8 Cl 20,4 N 16,3 S 9,4 %

Die Verbindung (f) entspricht dem alkylenverbrückten Guanidinothiazol (33) der Tabelle I.

## Patentansprüche

1. Alkylenverbrückte Guanidinothiazolderivate der allgemeinen Formel (I):

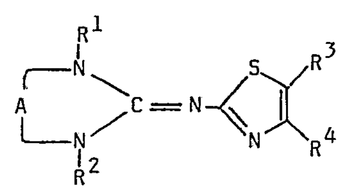

in welcher:

a) A für einen Ethylen, Rest,
$R^1$, $R^2$ und $R^3$ für Wasserstoff, und
$R^4$ für einen Methyl-, Chlormethyl-, Ethyloxycarbonylmethyl-, 5,6,7,8-Tetrahydro-2-naphthyl-, 4-Biphenylyl-, 4-Methoxyphenyl- und 4-Chlor-2-methylphenyl-Rest stehen, oder

b) A für einen Trimethylen-Rest,
$R^1$, $R^2$ und $R^3$ für Wasserstoff, und
$R^4$ für Wasserstoff, einen Methyl-, t.-Butyl-Chlormethyl-, Ethyloxycarbonylmethyl-, 2,4-Dichlorphenoxymethyl-, Phenylthiomethyl-, 1-Imidazolylmethyl-, 1,2,4-Triazol-2-yl-methyl-, Phenyl-, 4-Cyclohexylphenyl, 1-Naphthyl-, 5,6,7,8-Tetrahydro-2-naphthyl-, 5-Indanyl-, 2,4-Dichlorphenyl-, 4-Biphenylyl-, 4-(4'-Chlor)-biphenylyl-, 4-Chlor-2-methoxyphenyl-, 4-Hydroxyphenyl-, 4-Methoxyphenyl-, 4-Chlor-2-methylphenyl-, 4-(2',4'-Dichlor)-biphenylyl-, 4-Chlorphenyl-, 4-Nitrophenyl-, 2-Hydroxy-4-methoxyphenyl-, 5-Nitro-furan-2-yl, 2,6-Diethyl-4-hydroxyphenyl-, 4-Chlor-2-hydroxyphenyl-, 4-Aminophenyl-, 3-Acetylamino-4-chlor-2-methylphenyl-, 4-Chloracetylaminophenyl-, Adamantyl-, 1-Ethyloxycarbonyl-2-(2',6'-dichlorphenyl)-vinyl- und S-Isothioureidomethyl-Rest stehen, oder

c) A für einen Trimethylen-Rest,
$R^1$ und $R_2$ für Wasserstoff,
$R^4$ für Methyl, und
$R^3$ für einen Ethyloxycarbonyl- Cyclohexyloxycarbonyl-, 2-Acetyloxyethyl- und 4-Chlorphenylcarboxamid-Rest stehen, oder

d) A für einen Trimethylen-Rest,
$R^1$ und $R^3$ für Wasserstoff,
$R^2$ für Methyl, und
$R^4$ für einen Methyl-, Phenyl-, 4-Chlor-2-methylphenyl- und 2,4-Dichlorphenyl-Rest stehen, oder

e) A für einen Tetramethylenrest,
$R^1$, $R^2$ und $R^3$ für Wasserstoff, und
$R^4$ für einen Methyl-, Phenyl-, 4-Chlor-2-methylphenyl- und 2,4-Dichlorphenyl-Rest stehen, oder

f) A für einen Trimethylenrest,
$R^1$ und $R^2$ für Wasserstoff, und
$R^3$ und $R^4$ für Phenyl stehen, oder

g) A für einen Trimethylenrest,
$R^1$, $R^2$ und $R^3$ für Wasserstoff, und
$R^4$ für einen $N^1$-Cyan-$N^2$ -benzyl-$N^3$-phenylguanidin-Rest, einen $N^1$-Cyan-$N^2$-methyl-$N^3$-phenyl-guanidin-Rest und einen $N^1$-Cyan-$N^2$-phenyl-S-methyl-isothioharnstoff-Rest stehen und die Verknüpfung zum Thiazolring jeweils in der para-Position des Phenylrings erfolgt

sowie deren physiologisch verträgliche Salze mit anorganischen und organischen Säuren und Basen.

2. Alkylenverbrückte Guanidinothiazolderivate der allgemeinen Formel (I):

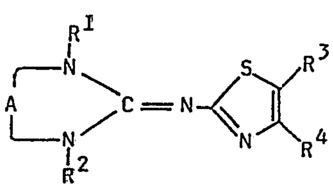

in welcher entweder:

a) A für einen Ethylen-Rest,
$R^1$, $R^2$ und $R^3$ für Wasserstoff, und
$R^4$ für einen Methyl-, Chlormethyl-, Ethyloxycarbonylmethyl-, 5,6,7,8-Tetrahydro-2-naphthyl-, 4-Biphenylyl-4-Methoxyphenyl- und 4-Chlor-2-methylphenyl-Rest stehen, oder

b) A für einen Trimethylen-Rest,
$R^1$, $R^2$ und $R^3$ für Wasserstoff, und
$R^4$ für Wasserstoff, einen Methyl-, t.-Butyl-, Chlormethyl-, Ethyloxycarbonylmethyl-, 2,4-Dichlorphenoxymethyl-, Phenylthiomethyl-, 1-Imidazolylmethyl, 1,2,4-Triazol-2-yl-methyl, Phenyl-, 4-Cyclohexylphenyl-, 1-Naphthyl-, 5,6,7,8-Tetrahydro-2-naphthlyl, 5-Indanyl-, 2,4-Dichlorphenyl-, 4-Biphenylyl-, 4-(4'-Chlor)-biphenylyl-, 4-Chlor-2-methoxyphenyl-, 4-Chlor-2-methylphenyl-, 4-(2',4'-Dichlor)-biphenylyl-, 4-chlorphenyl-, 4-Nitrophenyl-, 2-Hydroxy-4-methoxyphenyl-, 5-Nitro-furan-2-yl, 2,6-Diethyl-4-hydroxyphenyl-, 4-Chlor-2-hydroxyphenyl-, 4-Aminophenyl-, 3-Acetylamino-4-chlor-2-methylphenyl-, 4-Chloracetylaminophenyl-, Adamantyl-, 1-Ethyloxycarbonyl-2-(2',6'-dichlorphenyl)-vinyl- und S-Isothioureidomethyl-Rest stehen, oder

c) A für einen Trimethylen-Rest,
$R^1$ und $R^2$ für Wasserstoff,
$R^4$ für Methyl, und
$R^3$ für einen Ethyloxycarbonyl-, Cyclohexyloxycarbonyl-, 2-Acetyloxyethyl- und 4-Chlorphenylcarboxymid-Rest stehen,

d) A für einen Trimethylen-Rest,
$R^1$ und $R^3$ für Wasserstoff,
$R^2$ für Methyl, und
$R^4$ für einen Methyl-, Phenyl-, 4-Chlor-2-methylphenyl- und 2,4-Dichlorphenyl-Rest stehen, oder

e) A für einen Tetramethylenrest,
$R^1$, $R^2$ und $R^3$ für Wasserstoff, und
$R^4$ für einen Methyl-, Phenyl-, 4-Chlor-2-methylphenyl- und 2,4-Dichlorphenyl-Rest stehen, oder

f) A für einen Trimethylenrest,
$R^1$ und $R^2$ für Wasserstoff, und
$R^3$ und $R^4$ für Phenyl stehen, oder

g) A für einen Trimethylenrest,
$R^1$, $R^2$ und $R^3$ für Wasserstoff, und
$R^4$ für einen $N^1$-Cyan-$N^2$-benzyl-$N^3$-phenylguanidin-Rest, einen $N^1$-Cyan-$N^2$-methyl-$N^3$-phenyl-guanidin-Rest und einen $N^1$-Cyan-$N^2$-phenyl-S-methylisothioharnstoff-Rest stehen und die Verknüpfung zum Thiazolring jeweils in der para-Position des Phenylrings erfolgt,

sowie deren physiologisch verträgliche Salze mit anorganischen und organischen Säuren und Basen, zur Verwendung bei der Bekämpfung von Krankheiten des Arachidonsäure-Metabolismuszyklus.

3. Verwendung von alkylenverbrückten Guanidinthiazolderivaten der allgemeinen Formel (I):

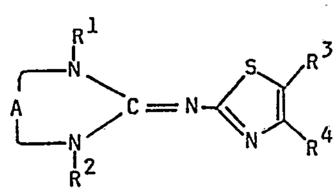

in welcher entweder:

a) A für einen Ethylen-Rest,
$R^1$, $R^2$ und $R^3$ für Wasserstoff, und
$R^4$ für einen Methyl-, Chlormethyl-, Ethyloxycarbonylmethyl-, 5,6,7,8-Tetrahydro-2-naphthyl-,

4-Biphenylyl-, 4-Methoxyphenyl- und 4-Chlor-2-methylphenyl-Rest stehen, oder
b) A für einen Trimethylen-Rest,
$R^1$, $R^2$ und $R^3$ für Wasserstoff, und
$R^4$ für Wasserstoff, einen Methyl-, t.-Butyl-, Chlormethyl-, Ethyloxycarbonylmethyl-, 2,4-Dichlorphenoxymethyl-, Phenylthiomethyl-, 1-Imidazolylmethyl-, 1,2,4-Triazol-2-yl-methyl-, Phenyl-, 4-Cyclohexylphenyl-, 1-Naphthyl-, 5,6,7,8-Tetrahydro-2-naphthyl-, 5-Indanyl-, 2,4-Dichlorphenyl-, 4-Biphenylyl-, 4-(4′-Chlor)-biphenylyl-, 4-Chlor-2-methoxyphenyl-, 4-Hydroxyphenyl-, 4-Methoxyphenyl-, 4-Chlor-2-methylphenyl-, 4-(2′,4′-Dichlor)-biphenylyl-, 4-Chlorphenyl-, 4-Nitrophenyl-, 2-Hydroxy-4-methoxyphenyl-, 5-Nitro-furan-2-yl-, 2,6-Diethyl-4-hydroxphenyl-, 4-Chlor-2-hydroxyphenyl-, 4-Aminophenyl-, 3-Acetylamino-4-chlor-2-methylphenyl-, 4-Chloracetylaminophenyl-, Adamantyl-, 1-Ethyloxycarbonyl-2-(2′,6′-dichlorphenyl)-vinyl- und S-Isothioureidomethyl-Rest stehen, oder
c) A für einen Trimethylen-Rest,
$R^1$ und $R^2$ für Wasserstoff,
$R^4$ für Methyl und
$R^3$ für einen Ethyloxycarbonyl-, Cyclohexyloxycarbonyl-, 2-Acetyloxyethyl- und 4-Chlorphenylcarboxamid-Rest stehen, oder
d) A für einen Trimethylen-Rest,
$R^1$ und $R^3$ für Wasserstoff,
$R^2$ für Methyl, und
$R^4$ für einen Methyl-, Phenyl-, 4-Chlor-2-methylphenyl- und 2,4-Dichlorphenyl-Rest stehen, oder
e) A für einen Tetramethylenrest,
$R^1$, $R^2$ und $R^3$ für Wasserstoff, und
$R^4$ für einen Methyl-, Phenyl-, 4-Chlor-2-methylphenyl- und 2,4-Dichlorphenyl-Rest stehen, oder
f) A für einen Trimethylenrest,
$R^1$ und $R^2$ für Wasserstoff, und
$R^3$ und $R^4$ für Phenyl stehen, oder
g) A für einen Trimethylenrest,
$R^1$, $R^2$ und $R^3$ für Wasserstoff, und
$R^4$ für einen $N^1$-Cyan-$N^2$-benzyl-$N^3$-phenyl-guanidin-Rest, einen $N^1$-Cyan-$N^2$-methyl-$N^3$-phenyl-guanidin-Rest und einen $N^1$-Cyan-$N^2$-phenyl-S-methyl-isothioharnstoff-Rest stehen und die Verknüpfung zum Thiazolring jeweils in der para-Position des Phenylrings erfolgt,
sowie deren physiologisch verträgliche Salze mit anorganischen und organischen Säuren und Basen, zur Zubereitung von Arzneimitteln zur Bekämpfung von Krankheiten des Arachidon-Metabolismuszyklus.

4. 2-(N(4′-(1″-Naphthyl)-thiazol-2′-yl)-imino)-hexahydropyrimidin der Formel:

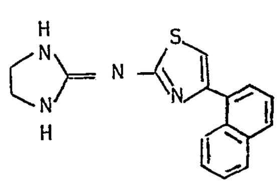

5. 2-(N-(4′-(2″,4″-Dichlorphenyl)-thiazol-2′-yl)-imino)-hexahydropyrimidin-hydrochlorid der Formel:

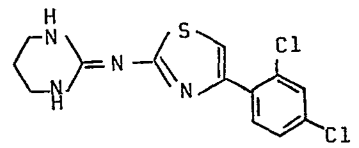

x HCl

6. 2-(N-(4′-Phenylthiazol-2′-yl)imino)-hexahydropyrimidinhydrochlorid der Formel:

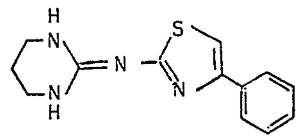

x HCl

7. Verfahren zur Herstellung von alkylenverbrückten Guanidinthiazolderivaten der allgemeinen Formel (I):

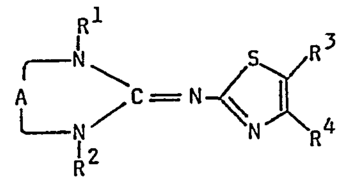

in welcher:
a) A für einen Ethylen-Rest,
$R^1$, $R^2$ und $R^3$ für Wasserstoff, und
$R^4$ für einen Methyl-, Chlormethyl-, Ethyloxycarbonylmethyl-, 5,6,7,8-Tetrahydro-2-naphthyl-, 4-Biphenylyl-, 4-Methoxyphenyl- und 4-Chlor-2-methylphenyl-Rest stehen, oder
b) A für einen Trimethylen-Rest,
$R^1$, $R^2$ und $R^3$ für Wasserstoff, und
$R^4$ für Wasserstoff, einen Methyl-, t.-Butyl-, Chlormethyl-, Ethyloxycarbonylmethyl-, 2,4-Dichlorphenoxymethyl-, Phenylthiomethyl-, 1-Imidazolylmethyl-, 1,2,4-Triazol-2-yl-methyl-, Phenyl-, 4-Cyclohexylphenyl-, 1-Naphthyl-, 5,6,7,8-Tetrahydro-2-naphthyl-, 5-Indanyl-, 2,4-Dichlorphenyl-, 4-Biphenylyl-, 4-(4′-Chlor)-biphenylyl-, 4-Chlor-2-methoxyphenyl-, 4-Hydroxyphenyl-, 4-Methoxyphenyl-, 4-Chlor-2-methylphenyl-, 4-(2′,4′-Dichlor)-biphenylyl-, 4-Chlorphenyl-, 4-Nitrophenyl-, 2-Hydroxy-4-methoxyphenyl-, 5-Nitro-furan-2-yl-, 2,6-Diethyl-4-hydroxyphenyl-, 4-Chlor-2-hydroxyphenyl-, 4-Aminophenyl-, 3-Acetylamino-4-chlor-2-methyl-, 4-Chloracetylaminophenyl-, Adamantyl-, 1-Ethyloxycarbonyl-2-(2′,6′-dichlorphenyl)-vinyl- und S-Isothioureidomethyl-Rest stehen, oder
c) A für einen Trimethylen-Rest,
$R^1$ und $R^2$ für Wasserstoff,
$R^4$ für Methyl, und
$R^3$ für einen Ethyloxycarbonyl-, Cyclohexyloxycarbonyl-, 2-Acetyloxyethyl- und 4-Chlorphenylcarboxamid-Rest stehen, oder
d) A für einen Trimethylen-Rest,
$R^1$ und $R^3$ für Wasserstoff,
$R^2$ für Methyl, und
$R^4$ für einen Methyl-, Phenyl-, 4-Chlor-2-methyl-phenyl- und 2,4-Dichlorphenyl-Rest stehen, oder

e) A für einen Tetramethylenrest,
$R^1$, $R^2$ und $R^3$ für Wasserstoff, und
$R^4$ für einen Methyl-, Phenyl-, 4-Chlor-2-methylphenyl- und 2,4-Dichlorphenyl-Rest stehen, oder

f) A für einen Trimethylenrest,
$R^1$ und $R^2$ für Wasserstoff, und
$R^3$ und $R^4$ für Phenyl stehen, oder

g) A für einen Trimethylenrest,
$R^1$, $R^2$ und $R^3$ für Wasserstoff, und
$R^4$ für einen $N^1$-Cyan-$N^2$-benzyl-$N^3$-phenylguanidin-Rest, einen $N^1$-Cyan-$N^2$-methyl-$N^3$-phenyl-guanidin-Rest und einen $N^1$-Cyan-$N^2$-phenyl-S-methylisothioharnstoff-Rest stehen und die Verknüpfung zum Thiazolring jeweils in der para-Position des Phenylrings erfolgt

sowie deren physiologisch verträgliche Salze mit anorganischen und organischen säuren und Basen,

dadurch gekennzeichnet, dass man gemäss nachstehendem Formelschema:

1. Alkylendiamine (II) mit N-Cyanimidodithio-Kohlensäure-5,5'-dimethylester (III) zu cyclischen N-Cyanguanidinen (IV) umsetzt,
2. diese mit Schwefelwasserstoff (V) zu N,N'-alkylenverbrückten Guanylthioharnstoffen (VI) in Gegenwart von Lösungsmitteln bei Temperaturen zwischen 0 bis 150°C umsetzt, und
3. diese (VI) mit α-Halogenketonen der α-Halogenaldehyden (VII) zu alkylenverbrückten Guanidinothiazolderivaten [(I) × HX] bzw. zu (I) nach Behandlung mit Basen umsetzt,

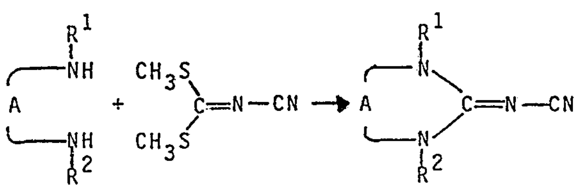

(II) (III) (IV)

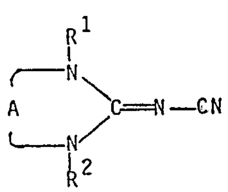

(IV)

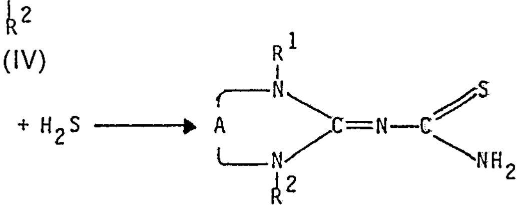

(V) (VI)

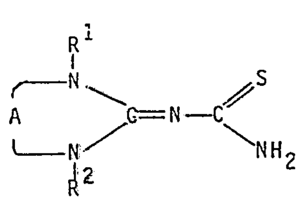

(VI)

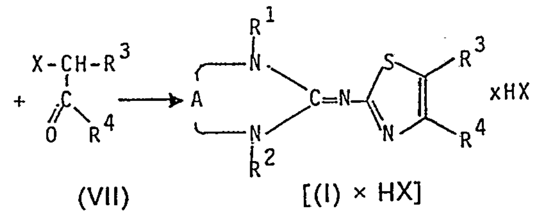

(VII) [(I) × HX]

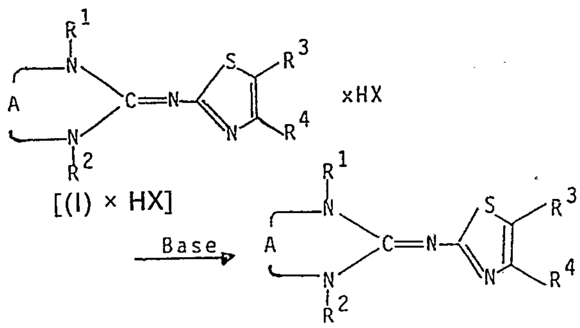


[(I) × HX]

(I)

wobei A, $R^1$, $R^2$, $R^3$ und $R^4$ die in Anspruch 1 angegebene Bedeutung haben.

## Claims

1. Alkylene-bridged guanidinothiazole derivatives of the general formula (I):

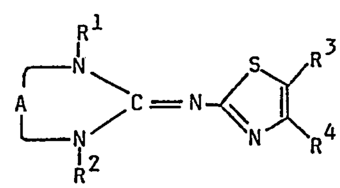

in which:

a) A represents an ethylene radical,
$R^1$, $R^2$ and $R^3$ represent hydrogen, and
$R^4$ represents a methyl, chloromethyl, ethyloxycarbonylmethyl, 5,6,7,8-tetrahydro-2-naphthyl, 4-biphenylyl, 4-methoxyphenyl and 4-chloro-2-methylphenyl radical, or

b) A represents a trimethylene radical,
$R^1$, $R^2$ and $R^3$ represent hydrogen, and
$R^4$ represents hydrogen, a methyl, t.-butyl, chloromethyl, ethyloxycarbonylmethyl, 2,4-dichlorophenoxymethyl, phenylthiomethyl, 1-imidazolylmethyl, 1,2,4-triazol-2-yl-methyl, phenyl, 4-cyclohexylphenyl, 1-naphthyl, 5,6,7,8-tetrahydro-2-naphthyl, 5-indanyl, 2,4-dichlorophenyl, 4-biphenylyl, 4-(4'-chloro)-biphenylyl, 4-chloro-2-methoxyphenyl, 4-hydroxyphenyl, 4-methoxyphenyl, 4-chloro-2-methylphenyl, 4-(2',4'-dichloro)-biphenylyl, 4-chlorophenyl, 4-nitrophenyl, 2-hydroxy-4-methoxyphenyl, 5-nitrofuran-2-yl, 2,6-diethyl-4-hydroxyphenyl, 4-chloro-2-hydroxyphenyl, 4-aminophenyl, 3-acetylamino-4-chloro-2-methylphenyl, 4-chloroacetylaminophenyl, adamantyl, 1-ethyloxycarbonyl-2-(2',6'-dichlorophenyl)-vinyl and S-isothioureidomethyl radical, or

c) A represents a trimethylene radical,
$R^1$ and $R^2$ represent hydrogen,
$R^4$ represents methyl, and
$R^3$ represents an ethyloxycarbonyl, cyclohexyloxycarbonyl, 2-acetyloxethyl and 4-chlorophenylcarboxamide radical, or
d) A represents a trimethylene radical,
$R^1$ and $R^3$ represent hydrogen,
$R^2$ represents methyl, and
$R^4$ represents a methyl, phenyl, 4-chloro-2-methylphenyl and 2,4-dichlorophenyl radical, or
e) A represents a tetramethylene radical,
$R^1$, $R^2$ and $R^3$ represent hydrogen, and
$R^4$ represents a methyl, phenyl, 4-chloro-2-methylphenyl and 2,4-dichlorophenyl radical, or
f) A represents a trimethylene radical,
$R^1$ and $R^2$ represent hydrogen, and
$R^3$ and $R^4$ represent phenyl, or
g) A represents a trimethylene radical,
$R^1$, $R^2$ and $R^3$ represent hydrogen, and
$R^4$ represents an $N^1$-cyano-$N^2$-benzyl-$N^3$-phenyl-guanidine radical, an $N^1$-cyano-$N^2$-methyl-$N^3$-phenyl-guanidine radical and an $N^1$-cyano-$N^2$-phenyl-S-methyl-isothiourea radical and the attachment to the thiazole ring is in each case in the para position of the phenyl ring,
and their physiologically tolerated salts with inorganic and organic acids and bases.

2. Alkylene-bridged guanidinothiazole derivatives of the general formula (I):

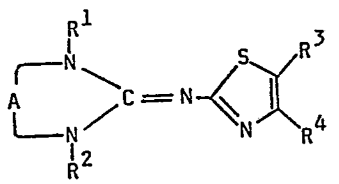

in which either:
a) A represents an ethylene radical,
$R^1$, $R^2$ and $R^3$ represent hydrogen, and
$R^4$ represents a methyl, chloromethyl, ethyloxycarbonylmethyl, 5,6,7,8-tetrahydro-2-naphthyl, 4-biphenylyl, 4-methoxyphenyl and 4-chloro-2-methylphenyl radical, or
b) A represents a trimethylene radical,
$R^1$, $R^2$ and $R^3$ represent hydrogen, and
$R^4$ represents hydrogen, a methyl, t.-butyl, chloromethyl, ethyloxycarbonylmethyl, 2,4-dichlorophenoxymethyl, phenylthiomethyl, 1-imidazolylmethyl, 1,2,4-triazol-2-yl-methyl, phenyl, 4-cyclohexylphenyl, 1-naphthyl, 5,6,7,8-tetrahydro-2-naphthyl, 5-indanyl, 2,4-dichlorophenyl, 4-biphenylyl, 4-(4'-chloro)-biphenylyl, 4-chloro-2-methoxyphenyl, 4-chloro-2-methylphenyl, 4-(2',4'-dichloro)-biphenylyl, 4-chlorophenyl, 4-nitrophenyl, 2-hydroxy-4-methoxyphenyl, 5-nitrofuran-2-yl, 2,6-diethyl-4-hydroxyphenyl, 4-chloror-2-hydroxyphenyl, 4-aminophenyl, 3-acetylamino-4-chloro-2-methylphenyl, 4-chloroacetylaminophenyl, adamantyl, 1-ethyloxycarbonyl-2-(2',6'-dichlorophenyl)-vinyl and S-isothioureidomethyl radical, or
c) A represents a trimethylene radical,
$R^1$ and $R^2$ represent hydrogen,
$R^4$ represents methyl, and
$R^3$ represents an ethyloxycarbonyl, cyclohexyloxycarbonyl, 2-acetyloxyethyl and 4-chlorophenylcarboxamide radical, or
d) A represents a trimethylene radical,
$R^1$ and $R^3$ represent hydrogen,
$R^2$ represents methyl, and
$R^4$ represents a methyl, phenyl, 4-chloro-2-methylphenyl and 2,4-dichlorophenyl radical, or
e) A represents a tetramethylene radical,
$R^1$, $R^2$ and $R^3$ represent hydrogen, and
$R^4$ represents a methyl, phenyl, 4-chloro-2-methylphenyl and 2,4-dichlorophenyl radical, or
f) A represents a trimethylene radical,
$R^1$ and $R^2$ represent hydrogen, and
$R^3$ and $R^4$ represent phenyl, or
g) A represents a trimethylene radical,
$R^1$, $R^2$ and $R^3$ represent hydrogen, and
$R^4$ represents an $N^1$-cyano-$N^2$-benzyl-$N^3$-phenyl-guanidine radical, an $N^1$-cyano-$N^2$-methyl-$N^3$-phenyl-guanidine radical and an $N^1$-cyano-$N^2$-phenyl-S-methyl-isothiourea radical and the attachment to the thiazole ring is in each case in the para position of the phenyl ring,
and their physiologically tolerated salts with inorganic and organic acids and bases, for use in combating disorders of the arachidonic acid metabolism cycle.

3. Use of alkylene-bridged guanidinothiazole derivatives of the general formula (I):

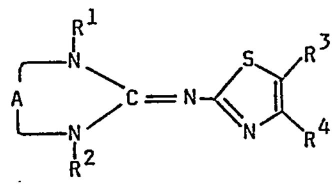

in which either:
a) A represents an ethylene radical,
$R^1$, $R^2$ and $R^3$ represent hydrogen, and
$R^4$ represents a methyl, chloromethyl, ethyloxycarbonylmethyl, 5,6,7,8-tetrahydro-2-naphthyl, 4-biphenylyl, 4-methoxyphenyl and 4-chloro-2-methylphenyl radical, or
b) A represents a trimethylene radical,
$R^1$, $R^2$ and $R^3$ represent hydrogen, and
$R^4$ represents hydrogen, a methyl, t.-butyl, chloromethyl, ethyloxycarbonylmethyl, 2,4-dichlorophenoxymethyl, phenylthiomethyl, 1-imidazolylmethyl, 1,2,4-triazol-2-yl-methyl, phenyl, 4-cyclohexylphenyl, 1-naphthyl, 5,6,7,8-tetrahydro-2-naphthyl, 5-indanyl, 2,4-dichlorophenyl, 4-biphenylyl, 4-(4'-chloro)-biphenylyl, 4-chloro-2-methoxyphenyl, 4-hydroxyphenyl, 4-methoxyphenyl, 4-chloro-2-methylphenyl, 4-(2',4'-dichloro)-biphenylyl, 4-chlorophenyl, 4-nitrophenyl, 2-hydroxy-4-methoxyphenyl, 5-nitrofuran-2-yl, 2,6-diethyl-4-hydroxyphenyl, 4-chloro-2-hydroxyphenyl, 4-aminophenyl, 3-acetylamino-4-chloro-2-methylphenyl, 4-chloroacetylaminophenyl, adamantyl, 1-ethyloxycarbonyl-2-(2',6'-dichlorophenyl)-vinyl and S-isothioureidomethyl radical, or
c) A represents a trimethylene radical,

$R^1$ and $R^2$ represent hydrogen,
$R^4$ represents methyl, and
$R^3$ represents an ethyloxycarbonyl, cyclohexyloxycarbonyl, 2-acetyloxyethyl and 4-chlorophenylcarboxamide radical, or
d) A represents a trimethylene radical,
$R^1$ and $R^3$ represent hydrogen,
$R^2$ represents methyl, and
$R^4$ represents a methyl, phenyl, 4-chloro-2-methylphenyl and 2,4-dichlorophenyl radical,
e) A represents a tetramethylene radical,
$R^1$, $R^2$ and $R^3$ represent hydrogen, and
$R^4$ represents a methyl, phenyl, 4-chloro-2-methylphenyl and 2,4-dichlorophenyl radical, or
f) A represents a trimethylene radical,
$R^1$ and $R^2$ represent hydrogen, and
$R^3$ and $R^4$ represent phenyl, or
g) A represents a trimethylene radical,
$R^1$, $R^2$ and $R^3$ represent hydrogen, and
$R^4$ represents an $N^1$-cyano-$N^2$-benzyl-$N^3$-phenyl-guanidine radical, an $N^1$-cyano-$N^2$-methyl-$N^3$-phenyl-guanidine radical and an $N^1$-cyano-$N^2$-phenyl-S-methyl-isothiourea radical and the attachment to the thiazole ring is in each case in the para position of the phenyl ring,
and their physiologically tolerated salts with inorganic and organic acids and bases, for preparing medicaments for combating disorders of the arachidonic acid metabolism cycle.

4. 2-(N(4'-(1''-naphthyl)-thiazol-2'-yl)-imino)-hexahydropyrimidine of the formula:

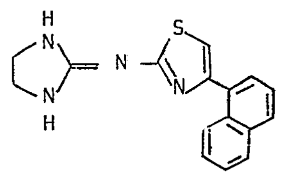

5. 2-(N-(4'-(2'',4''-dichlorophenyl)-thiazol-2'-yl)-imino)-hexahydropyrimidine hydrochloride of the formula:

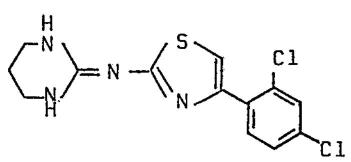

x HCl

6. 2-(N-(4'-phenylthiazol-2'-yl)imino)-hexahydropyrimidine hydrochloride of the formula:

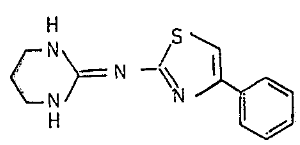

x HCl

7. Process for the preparation of alkylene-bridged guanidinothiazole derivatives of the general formula (I):

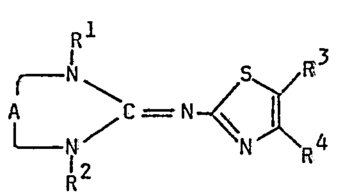

in which:
a) A represents a ethylene radical,
$R^1$, $R^2$ and $R^3$ represent hydrogen, and
$R^4$ represents a methyl, chloromethyl, ethyloxycarbonylmethyl, 5,6,7,8-tetrahydro-2-naphthyl, 4-biphenylyl, 4-methoxyphenyl and 4-chloro-2-methylphenyl radical, or
b) A represents a trimethylene radical,
$R^1$, $R^2$ and $R^3$ represent hydrogen, and
$R^4$ represents hydrogen, a methyl, t.-butyl, chloromethyl, ethyloxycarbonylmethyl, 2,4-dichlorophenoxymethyl, phenylthiomethyl, 1-imidazolylmethyl, 1,2,4-triazol-2-yl-methyl, phenyl, 4-cyclohexylphenyl, 1-naphthyl, 5,6,7,8-tetrahydro-2-naphthyl, 5-indanyl, 2,4-dichlorophenyl, 4-biphenylyl, 4-(4'-chloro)-biphenylyl, 4-chloro-2-methoxyphenyl, 4-hydroxyphenyl, 4-methoxyphenyl, 4-chloro-2-methylphenyl, 4-(2',4'-dichloro)-biphenylyl, 4-chlorophenyl, 4-nitrophenyl, 2-hydroxy-4-methoxyphenyl, 5-nitrofuran-2-yl, 2,6-diethyl-4-hydroxyphenyl, 4-chloro-2-hydroxyphenyl, 4-aminophenyl, 3-acetylamino-4-chloro-2-methyl-, 4-chloro-acetylaminophenyl, adamantyl, 1-ethyloxycarbonyl-2-(2',6'-dichlorophenyl)-vinyl and S-isothioureidomethyl radical, or
c) A represents a trimethylene radical,
$R^1$ and $R^2$ represent hydrogen,
$R^4$ represents methyl, and
$R^3$ represents an ethyloxycarbonyl, cyclohexyloxycarbonyl, 2-acetyloxyethyl and 4-chlorophenylcarboxamide radical, or
d) A represents a trimethylene radical,
$R^1$ and $R^3$ represent hydrogen,
$R^2$ represents methyl, and
$R^4$ represents a methyl, phenyl, 4-chloro-2-methylphenyl and 2,4-dichlorophenyl radical, or
e) A represents a tetramethylene radical,
$R^1$, $R^2$ and $R^3$ represent hydrogen, and
$R^4$ represents a methyl, phenyl, 4-chloro-2-methylphenyl and 2,4-dichlorophenyl radical, or
f) A represents a trimethylene radical,
$R^1$ and $R^2$ represent hydrogen, and
$R^3$ and $R^4$ represent phenyl, or
g) A represents a trimethylene radical,
$R^1$, $R^2$ and $R^3$ represent hydrogen, and
$R^4$ represents an $N^1$-cyano-$N^2$-benzyl-$N^3$-phenyl-guanidine radical, an $N^1$-cyano-$N^2$-methyl-$N^3$-phenyl-guanidine radical and an $N^1$-cyano-$N^2$-phenyl-S-methylisothiourea radical and the attachment to the thiazole ring is in each case in the para position of the phenyl ring,
and their physiologically tolerated salts with inorganic acids and bases, characterised in that, according to the equation below:

1. alkylenediamines (II) are reacted with N-cyanimidothiocarbonic acid 5,5'-dimethyl ester (III) to give cyclic N-cyanoguanidines (IV),
2. these are reacted with hydrogen sulphide (V) in the presence of solvents at temperatures between 0 and 150°C to give N,N'-alkylene-bridged guanylthioureas (VI), and
3. these (VI) are reacted with α-halogenoketones of the α-halogenoaldehydes (VII) to give

alkylene-bridged guanidinothiazole derivatives [(I) × HX] or to give (I) after treatment with bases:

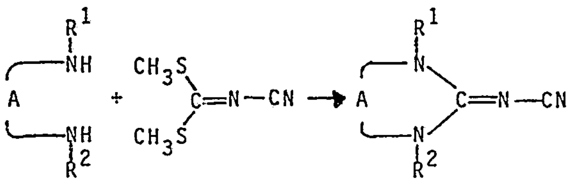

(II) (III) (IV)

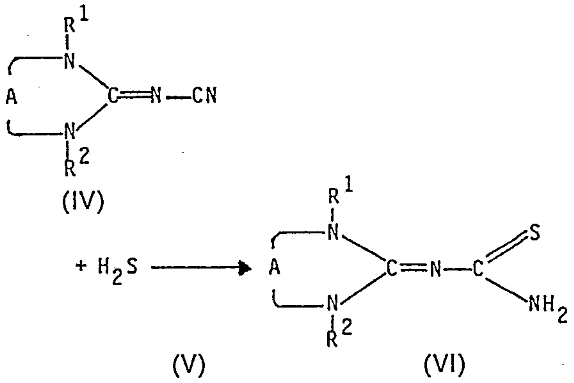

(IV)

(V) (VI)

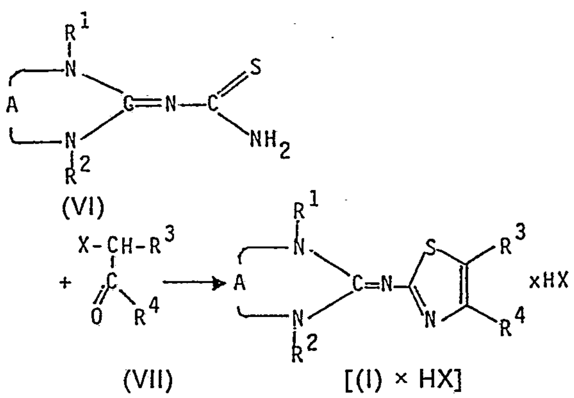

(VI)

(VII) [(I) × HX]

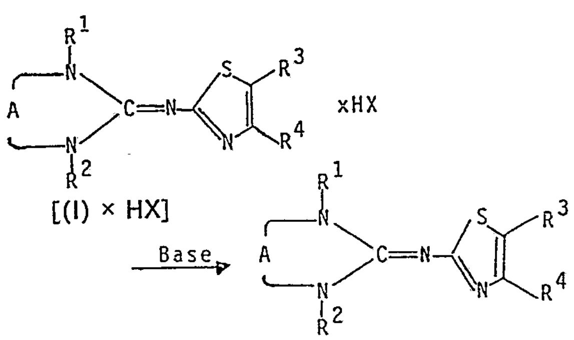


[(I) × HX]

(I)

wherein:

A, $R^1$, $R^2$, $R^3$ and $R^4$ have the meaning given in Claim 1.

## Revendications

1. Dérivés de guanidinothiazole à pont alkylène, de formule générale (I):

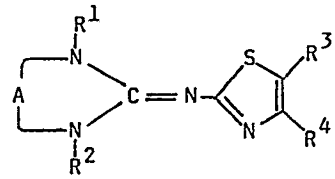

dans laquelle:

a) A représente un reste éthylène,

$R^1$, $R^2$ et $R^3$ représentent un atome d'hydrogène, et

$R^4$ représente un reste méthyle, chlorométhyle, éthyloxycarbonylméthyle, 5,6,7,8-tétrahydro-2-naphtyle, 4-biphénylyle, 4-méthoxyphényle et 4-chloro-2-méthylphényle, ou

b) A représente un reste triméthylène,

$R^1$, $R^2$ et $R^3$ représentent chacun un atome d'hydrogène, et

$R^4$ représente un atome d'hydrogène, un reste méthyle, t.-butyle, chlorométhyle, éthyloxycarbonylméthyle, 2,4-dichlorophénoxyméthyle, phénylthiométhyle, 1-imidazolylméthyle, 1,2,4-triazol-2-yl-méthyle, phényle, 4-cyclohexyl-phényle, 1-naphtyle, 5,6,7,8-tétrahydro-2-naphtyle, 5-indanyle, 2,4-dichlorophényle, 4-biphénylyle, 4-(4'-chloro)-biphénylyle, 4-chloro-2-méthoxyphényle, 4-hydroxyphényle, 4-méthoxyphényle, 4-chloro-2-méthylphényle, 4-(2',4'-dichloro)biphénylyle, 4-chlorophényle, 4-nitrophényle, 2-hydroxy-4-méthoxyphényle, 5-nitro-furanne-2-yle, 2,6-diéthyl-4-hydroxyphényle, 4-chloro-2-hydroxyphényle, 4-aminophényle, 3-acétylamino-4-chloro-2-méthylphényle, 4-chloroacétylaminophényle, adamantyle, 1-éthyloxycarbonyl-2-(2',6'-dichlorophényl)-vinyle et S-isothio-uréidométhyle, ou

c) A représente un reste triméthylène,

$R^1$ et $R^2$ représentent chacun un atome d'hydrogène,

$R^4$ représente un reste méthyle, et

$R^3$ représente un reste éthyloxycarbonyle, cyclohexyloxycarbonyle, 2-acétyloxyéthyle et 4-chlorophénylcarboxamide, ou

d) A représente un reste triméthylène,

$R^1$ et $R^3$ représentent chacun un atome d'hydrogène,

$R^2$ représente un groupe méthyle, et

$R^4$ représente un reste méthyle, phényle, 4-chloro-2-méthylphényle et 2,4-dichlorophényle, ou

e) A représente un reste tétraméthylène,

$R^1$, $R^2$ et $R^3$ représentent chacun un atome d'hydrogène, et

$R^4$ représente un reste méthyle, phényle, 4-chloro-2-méthylphényle et 2,4-dichlorophényle, ou

f) A représente un reste triméthylène,

$R^1$ et $R^2$ représentent un atome d'hydrogène, et

$R^3$ et $R^4$ représentent un reste phényle, ou

g) A représente un reste triméthylène,

$R^1$, $R^2$ et $R^3$ représentent chacun un atome d'hydrogène et

$R^4$ représente un reste $N^1$-cyano-$N^2$-benzyl-$N^3$-phényl-guanidine, un reste $N^1$-cyano-$N^2$-

méthyl-$N^3$-phényl-guanidine et un reste $N^1$-cyano-$N^2$-phényl-S-méthyl-isothio-urée, et

la liaison au cycle thiazole s'effectue toujours en position para du cycle phényle, ainsi que leurs sels physiologiquement acceptables avec des acides et bases minéraux et organiques.

2. Dérivés de guanidinothiazole à pont alkylène, de formule générale (I):

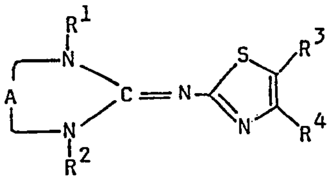

dans laquelle:

a) A représente un reste éthylène,

$R^1$, $R^2$ et $R^3$ représentent chacun un atome d'hydrogène, et

$R^4$ représente un reste méthyle, chlorométhyle, éthyloxycarbonylméthyle, 5,6,7,8,-tétrahydro-2-naphtyle, 4-biphénylyl-4-méthoxyphényle et 4-chloro-2-méthylphényle, ou

b) A représente un reste triméthylène,

$R^1$, $R^2$ et $R^3$ représentent chacun un atome d'hydrogène, et

$R^4$ représente un atome d'hydrogène, un reste méthyle, t.-butyle, chlorométhyle, éthyloxycarbonylméthyle, 2,4-dichlorophénoxyméthyle, phénylthiométhyle, 1-imidazolylméthyle, 1,2,4-triazol-2-yl-méthyle, phényle, 4-cyclohexylphényle, 1-naphtyle, 5,6,7,8-tétrahydro-2-naphtyle, 5-indanyle, 2,4-dichlorophényle, 4-biphénylyle, 4-(4'-chloro)-biphénylyle, 4-chloro-2-méthoxyphényle, 4-chloro-2-méthyl-phényle, 4-(2',4'-dichloro)-biphénylyle, 4-chlorophényle, 4-nitrophényle, 2-hydroxy-4-méthoxyphényle, 5-nitro-furanne-2-yle, 2,6-diéthyl-4-hydroxyphényle, 4-chloro-2-hydroxyphényle, 4-aminophényle, 3-acétylamino-4-chloro-2-méthylphényle, 4-chloroacétylaminophényle, adamantyle, 1-éthyloxycarbonyl-2-(2',6'-dichlorophényl)-vinyle et S-isothio-uréidométhyle, ou

c) A représente un reste triméthylène,

$R^1$ et $R^2$ représentent chacun un atome d'hydrogène,

$R^4$ représente un reste méthyle, et

$R^3$ représente un reste éthyloxycarbonyle, cyclohexyloxycarbonyle, 2-acétyloxyéthyle et 4-chlorophénylcarboxamide, ou

d) A représente un reste triméthylène,

$R^1$ et $R^3$ représentent un atome d'hydrogène,

$R^2$ représente un reste méthyle, et

$R^4$ représente un reste méthyle, phényle, 4-chloro-2-méthylphényle et 2,4-dichlorophényle, ou

e) A représente un reste tétraméthylène,

$R^1$, $R^2$ et $R^3$ représentent un atome d'hydrogène, et

$R^4$ représente un reste méthyle, phényle, 4-chloro-2-méthylphényle et 2,4-dichlorophényle, ou

f) A représente un reste triméthylène,

$R^1$ et $R^2$ représentent un atome d'hydrogène, et $R^3$ et $R^4$ représentent un reste phényle, ou

g) A représente un reste triméthylène,

$R^1$, $R^2$ et $R^3$ représentent chacun un atome d'hydrogène et

$R^4$ représente un reste $N^1$-cyano-$N^2$-benzyl-$N^3$-phényl-guanidine, un reste $N^1$-cyano-$N^2$-méthyl-$N^3$-phényl-guanidine et un reste $N^1$-cyano-$N^2$-phényl-S-méthyl-isothio-urée, et

la liaison au noyau thiazole s'effectue toujours en position para du noyau phényle,

ainsi que leurs sels physiologiquement acceptables avec des acides et bases minéraux et organiques, à utiliser pour combattre les maladies du cycle de métabolisme de l'acide arachidonique.

3. Utilisation de dérivés de guanidinothiazole, à pont alkylène, de formule générale (I):

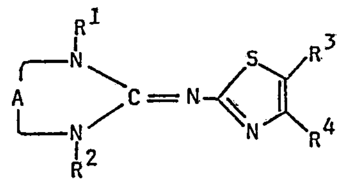

dans laquelle:

a) A représente un reste éthylène,

$R^1$, $R^2$ et $R^3$ représentent un atome d'hydrogène, et

$R^4$ représente un reste méthyle, chlorométhyle, éthyloxycarbonylméthyle, 5,6,7,8-tétrahydro-2-naphtyle, 4-biphénylyle, 4-méthoxyphényle et 4-chloro-2-méthylphényle, ou

b) A représente un reste triméthylène,

$R^1$, $R^2$ et $R^3$ représentent chacun un atome d'hydrogène, et

$R^4$ représente un atome d'hydrogène, un reste méthyle, t.-butyle, chlorométhyle, éthyloxycarbonylméthyle, 2,4-dichlorophénoxyméthyle, phénylthiométhyle, 1-imidazolylméthyle, 1,2,4-triazol-2-yl-méthyle, phényle, 4-cyclohexyl-phényle, 1-naphtyle, 5,6,7,8-tétrahydro-2-naphtyle, 5-indanyle, 2,4-dichlorophényle, 4-biphénylyle, 4-(4'-chloro)-biphénylyle, 4-chloro-2-méthoxyphényle, 4-hydroxyphényle, 4-méthoxyphényle, 4-chloro-2-méthylphényle, 4-(2',4'-dichloro)-biphénylyle, 4-chlorophényle, 4-nitrophényle, 2-hydroxy-4-méthoxyphényle, 5-nitro-furanne-2-yle, 2,6-diéthyl-4-hydroxyphényle, 4-chloro-2-hydroxyphényle, 4-aminophényle, 3-acétylamino-4-chloro-2-méthylphényle, 4-chloroacétylaminophényle, adamantyle, 1-éthyloxycarbonyl-2-(2',6'-dichlorophényl)-vinyle et S-isothio-uréidométhyle,

ou

c) A représente un reste triméthylène,

$R^1$ et $R^2$ représentent chacun un atome d'hydrogène,

$R^4$ représente un reste méthyle, et

$R^3$ représente un reste éthyloxycarbonyle, cyclohexyloxycarbonyle, 2-acétyloxyéthyle et 4-chlorophénylcarboxamide, ou

d) A représente un reste triméthylène,

$R^1$ et $R^3$ représentent chacun un atome d'hydrogène,

$R^2$ représente un groupe méthyle, et

$R^4$ représente un reste méthyle, phényle, 4-chloro-2-méthylphényle et 2,4-dichlorophényle, ou

e) A représente un reste tétraméthylène,

$R^1$, $R^2$ et $R^3$ représentent chacun un atome d'hydrogène, et

$R^4$ représente un reste méthyle, phényle, 4-chloro-2-méthylphényle et 2,4-dichlorophényle, ou

f) A représente un reste triméthylène,

$R^1$ et $R^2$ représentent un atome d'hydrogène, et

$R^3$ et $R^4$ représentent un reste phényle, ou

g) A représente un reste triméthylène,

$R^1$, $R^2$ et $R^3$ représentent chacun un atome d'hydrogène, et

$R^4$ représente un reste $N^1$-cyano-$N^2$-benzyl-$N^3$-phényl-guanidine, un reste $N^1$-cyano-$N^2$-méthyl-$N^3$-phényl-guanidine et un reste $N^1$-cyano-$N^2$-phényl-S-méthyl-isothio-urée, et

la liaison au cycle thiazole s'effectue toujours en position para du cycle phényle,

ainsi que leurs sels physiologiquement acceptables avec des acides et bases minéraux et organiques, pour préparer des médicaments pour combattre des maladies du cycle de métabolisme de l'acide arachidonique.

4. 2-(N-(4'-(1"-naphtyl)-thiazol-2'-yl)-imino)-hexahydropyrimidine de formule:

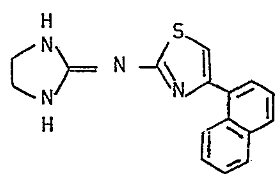

5. Chlorhydrate de 2-(N-(4'-(2",4"-dichlorophényl)-thiazol-2'-yl)-imino)-hexahydropyrimidine de formule:

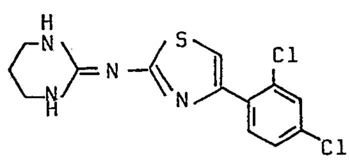

x HCl

6. Chlorhydrate de 2-(N-(4'-phénylthiazol-2'-yl)imino)-hexahydropyrimidine de formule:

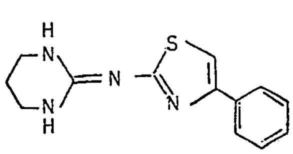

x HCl

7. Procédé de préparation de dérivés de guanidinothiazole, à pont alkylène, de formule générale (I):

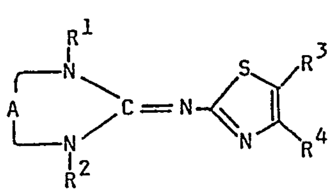

dans laquelle:

a) A représente un reste éthylène,

$R^1$, $R^2$ et $R^3$ représentent un atome d'hydrogène, et

$R^4$ représente un reste méthyle, chlorométhyle, éthyloxycarbonylméthyle, 5,6,7,8-tétrahydro-2-naphtyle, 4-biphénylyle, 4-méthoxyphényle et 4-chloro-2-méthylphényle, ou

b) A représente un reste triméthylène,

$R^1$, $R^2$ et $R^3$ représentent chacun un atome d'hydrogène, et

$R^4$ représente un atome d'hydrogène, un reste méthyle, t.-butyle, chorométhyle, éthyloxycarbonylméthyle, 2,4-dichlorophénoxyméthyle, phénylthiométhyle, 1-imidazolylméthyle, 1,2,4-triazol-2-yl-méthyle, phényle, 4-cyclohexyl-phényle, 1-naphtyle, 5,6,7,8-tétrahydro-2-naphtyle, 5-indanyle, 2,4-dichlorophényle, 4-biphénylyle, 4-(4'-chloro)-biphénylyle, 4-chloro-2-méthoxyphényle, 4-hydroxyphényle, 4-méthoxyphényle, 4-chloro-2-méthylphényle, 4-(2',4'-dichloro)-biphénylyle, 4-chlorophényle, 4-nitrophényle, 2-hydroxy-4-méthoxyphényle, 5-nitro-furanne-2-yle, 2,6-diéthyl-4-hydroxyphényle, 4-chloro-2-hydroxyphényle, 4-aminophényle, 3-acétylamino-4-chloro-2-méthylméthyle, 4-chloroacétylaminophényle, adamantyle, 1-éthyloxycarbonyl-2-(2',6'-dichlorophényl)-vinyle et S-isothio-uréidométhyle, ou

c) A représente un reste triméthylène,

$R^1$ et $R^2$ représentent chacun un atome d'hydrogène,

$R^4$ représente un reste méthyle, et

$R^3$ représente un reste éthyloxycarbonyle, cyclohexyloxycarbonyle, 2-acétyloxyéthyle et 4-chlorophénylcarboxamide, ou

d) A représente un reste triméthylène,

$R^1$ et $R^3$ représentent chacun un atome d'hydrogène,

$R^2$ représente un groupe méthyle, et

$R^4$ représente un reste méthyle, phényle, 4-chloro-2-méthylphényle et 2,4-dichlorophényle, ou

e) A représente un reste tétraméthylène,

$R^1$, $R^2$ et $R^3$ représentent chacun un atome d'hydrogène, et

$R^4$ représente un reste méthyle, phényle, 4-chloro-2-méthylphényle et 2,4-dichlorophényle, ou

f) A représente un reste triméthylène,

$R^1$ et $R^2$ représentent un atome d'hydrogène, et

$R^3$ et $R^4$ représentent un reste phényle, ou

g) A représente un reste triméthylène,

$R^1$, $R^2$ et $R^3$ représentent chacun un atome d'hydrogène, et

$R^4$ représente un reste $N^1$-cyano-$N^2$-benzyl-$N^3$-phényl-guanidine, un reste $N^1$-cyano-$N^2$-méthyl-$N^3$-phényl-guanidine et un reste $N^1$-cyano-$N^2$-phényl-S-méthyl-isothio-urée, et

la liaison au cycle thiazole s'effectue toujours en position para du cycle phényle,

ainsi que leurs sels physiologiquement acceptables avec des acides et bases minéraux et organiques,

caractérisé en ce que, selon le schéma suivant des formules:

1. on fait réagir des alkylènediamines (II) avec l'ester 5,5'-diméthylique de l'acide N-cyanimido-dithio-carboxylique (III) pour obtenir les N-cyanoguanidines cycliques (IV);

2. on fait réagir ces dernières avec l'hydrogène sulfuré (V) pour obtenir des guanylthio-urées à pont N,N'-alkylène (VI), en présence de solvants à des températures comprises entre 0 et 150°C, et

3. on fait réagir ce composé (VI) avec des α-halogénocétones ou des α-halogénoaldéhydes (VII) pour obtenir des dérivés de guanidinothiazole à pont alkylène [(I) × HX] ou pour obtenir (I) après traitement par des bases:

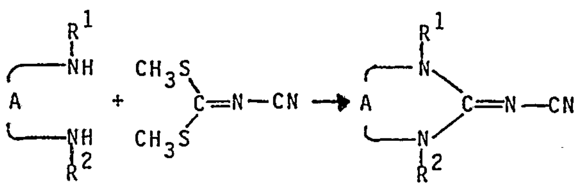

(II) (III) (IV)

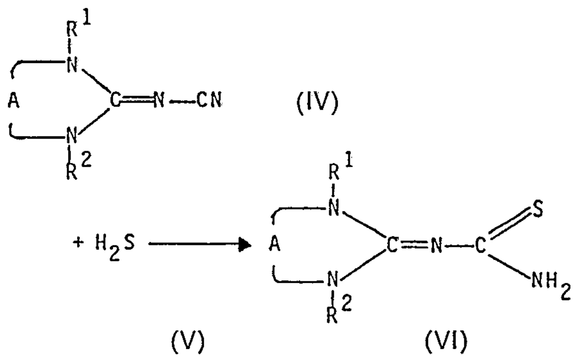

(V) (VI)

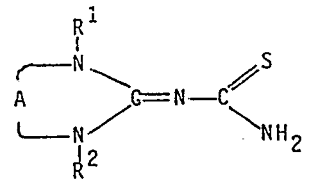

(VI)

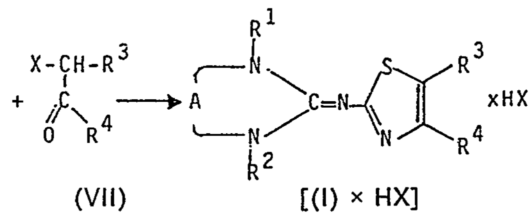

(VII) [(I) × HX]

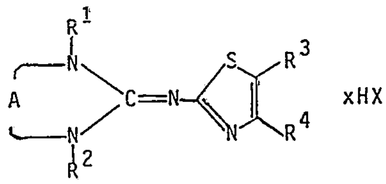

[(I) × HX]

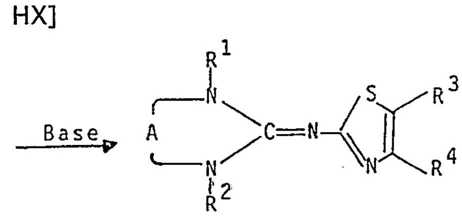

(I)

où A, R¹, R², R³ et R⁴ ont le sens indiqué à la revendication 1.